(19) Europäisches Patentamt

European Patent Office

Office européen des brevets

(11) **EP 1 538 212 A1**

(12) **EUROPÄISCHE PATENTANMELDUNG**

(43) Veröffentlichungstag:
**08.06.2005 Patentblatt 2005/23**

(51) Int Cl.⁷: **C12N 15/54**, C12N 9/12,
C12N 1/21, G01N 33/68

(21) Anmeldenummer: **04027608.1**

(22) Anmeldetag: **20.11.2004**

(84) Benannte Vertragsstaaten:
**AT BE BG CH CY CZ DE DK EE ES FI FR GB GR
HU IE IS IT LI LU MC NL PL PT RO SE SI SK TR**
Benannte Erstreckungsstaaten:
**AL HR LT LV MK YU**

(30) Priorität: **03.12.2003 DE 10356433**

(71) Anmelder: **Bayer CropScience AG
40789 Monheim (DE)**

(72) Erfinder:
• **Wieczorke, Roman, Dr.
40627 Düsseldorf (DE)**
• **Suelmann, Rüdiger, Dr.
50733 Köln (DE)**
• **Schreier, Peter, Prof. Dr.
50674 Köln (DE)**
• **Leuthner, Birgitta, Dr.
40764 Langenfeld (DE)**

(54) **Verfahren zum Identifizieren von fungizid wirksamen Verbindungen basierend auf UMP/CMP-Kinasen aus Pilzen**

(57) Die Erfindung bezieht sich auf ein Verfahren zum Identifizieren von Fungiziden, die Verwendung einer UMP/CMP-Kinase zum Identifizieren von Fungiziden, sowie auf Nukleinsäuren kodierend für UMP/CMP-Kinasen und die davon kodierten Polypeptide.

**EP 1 538 212 A1**

**SEQUENCE LISTING**

<110>  Bayer CropScience AG

<120>   Verfahren zum Identifizieren von fungizid wirksamen Verbindungen basierend auf UMP/CMP-Kinasen aus Pilzen

<130>  BCS 03-3088

<160>  2

<170>  PatentIn version 3.1

<210>  1
<211>  798
<212>  DNA
<213>  Ustilago maydis

<220>
<221>  CDS
<222>  (1)..(798)
<223>

<400>  1

```
atg ggt ctg ttc gac aag ctc aag cac aag aag cac gaa ccg gtc gag     48
Met Gly Leu Phe Asp Lys Leu Lys His Lys Lys His Glu Pro Val Glu
1               5                   10                  15

gca gct cct gcc cct gct gct cac att gca tct gca ccc gct gct gat     96
Ala Ala Pro Ala Pro Ala Ala His Ile Ala Ser Ala Pro Ala Ala Asp
                20                  25                  30

gct gag act ggt gct ggt gcc gtt gag aag gat acg cca cga ttc gac    144
Ala Glu Thr Gly Ala Gly Ala Val Glu Lys Asp Thr Pro Arg Phe Asp
        35                  40                  45

tcg tcc aaa gtg acg gtc gtc ttt gtg ctt ggc gga ccc ggt gct ggc    192
Ser Ser Lys Val Thr Val Val Phe Val Leu Gly Gly Pro Gly Ala Gly
    50                  55                  60

aaa ggc acc cag tgt gct cgt ctc gta caa gat tac ggc ttt gtt cac    240
Lys Gly Thr Gln Cys Ala Arg Leu Val Gln Asp Tyr Gly Phe Val His
65                  70                  75                  80
```

ctt agc gcc ggt gat ctg ctg cgt gcc gaa cag caa cgt cca ggc tcc    288
Leu Ser Ala Gly Asp Leu Leu Arg Ala Glu Gln Gln Arg Pro Gly Ser
            85              90            95

cag tac ggt gcc atg atc gcc gac tac atc aag gaa ggc aag atc gtg    336
Gln Tyr Gly Ala Met Ile Ala Asp Tyr Ile Lys Glu Gly Lys Ile Val
            100          105          110

ccc atg gaa gta acc gtc gct ctg ctt tcc aac gcg atc gcg gaa gct    384
Pro Met Glu Val Thr Val Ala Leu Leu Ser Asn Ala Ile Ala Glu Ala
            115          120          125

ctc tcc aag caa gcc act acc gaa gcc gat cac tcg atc cct gaa gag    432
Leu Ser Lys Gln Ala Thr Thr Glu Ala Asp His Ser Ile Pro Glu Glu
            130          135          140

cac aaa ctc aaa tgg agc gat ggc aag ggt cgc ttc ctt gtc gac ggc    480
His Lys Leu Lys Trp Ser Asp Gly Lys Gly Arg Phe Leu Val Asp Gly
145            150          155          160

ttc cca cgc aag atg gac cag gcg atc aaa ttt gac gaa tca gta tgc    528
Phe Pro Arg Lys Met Asp Gln Ala Ile Lys Phe Asp Glu Ser Val Cys
            165          170          175

gag tcc aag ttt gtg ctg ttc ctg cag tgc agt gaa gaa gtg atg ctc    576
Glu Ser Lys Phe Val Leu Phe Leu Gln Cys Ser Glu Glu Val Met Leu
            180          185          190

gag aga ttg ctc gag cgt gga aag acc agc ggt cgc gcc gat gac aac    624
Glu Arg Leu Leu Glu Arg Gly Lys Thr Ser Gly Arg Ala Asp Asp Asn
            195          200          205

atc gag tcg atc aag aag cgt ttc cag acc ttt gta gag acc tcg atg    672
Ile Glu Ser Ile Lys Lys Arg Phe Gln Thr Phe Val Glu Thr Ser Met
            210          215          220

cct gtg gtc gac tac tac cga aaa cag gac cgc gtg gta gag gtc gac    720
Pro Val Val Asp Tyr Tyr Arg Lys Gln Asp Arg Val Val Glu Val Asp
225            230          235          240

tcg atc aag acg gtg gat cag gtc tat gcc gag atc aag gag gct atg    768
Ser Ile Lys Thr Val Asp Gln Val Tyr Ala Glu Ile Lys Glu Ala Met
            245          250          255

'

```
gat cgc acc ttt gcc aag ctt gag cag tag                                    798
Asp Arg Thr Phe Ala Lys Leu Glu Gln
            260             265


<210>  2
<211>  265
<212>  PRT
<213>  Ustilago maydis

<400>  2

Met Gly Leu Phe Asp Lys Leu Lys His Lys Lys His Glu Pro Val Glu
1               5               10              15


Ala Ala Pro Ala Pro Ala Ala His Ile Ala Ser Ala Pro Ala Ala Asp
            20              25              30


Ala Glu Thr Gly Ala Gly Ala Val Glu Lys Asp Thr Pro Arg Phe Asp
            35              40              45


Ser Ser Lys Val Thr Val Val Phe Val Leu Gly Gly Pro Gly Ala Gly
        50              55              60


Lys Gly Thr Gln Cys Ala Arg Leu Val Gln Asp Tyr Gly Phe Val His
65              70              75              80


Leu Ser Ala Gly Asp Leu Leu Arg Ala Glu Gln Gln Arg Pro Gly Ser
            85              90              95


Gln Tyr Gly Ala Met Ile Ala Asp Tyr Ile Lys Glu Gly Lys Ile Val
            100             105             110


Pro Met Glu Val Thr Val Ala Leu Leu Ser Asn Ala Ile Ala Glu Ala
            115             120             125


Leu Ser Lys Gln Ala Thr Thr Glu Ala Asp His Ser Ile Pro Glu Glu
        130             135             140


His Lys Leu Lys Trp Ser Asp Gly Lys Gly Arg Phe Leu Val Asp Gly
145             150             155             160


Phe Pro Arg Lys Met Asp Gln Ala Ile Lys Phe Asp Glu Ser Val Cys
                165             170             175
```

```
Glu Ser Lys Phe Val Leu Phe Leu Gln Cys Ser Glu Glu Val Met Leu
            180                 185                 190

Glu Arg Leu Leu Glu Arg Gly Lys Thr Ser Gly Arg Ala Asp Asp Asn
            195                 200                 205

Ile Glu Ser Ile Lys Lys Arg Phe Gln Thr Phe Val Glu Thr Ser Met
        210                 215                 220

Pro Val Val Asp Tyr Tyr Arg Lys Gln Asp Arg Val Val Glu Val Asp
    225                 230                 235                 240

Ser Ile Lys Thr Val Asp Gln Val Tyr Ala Glu Ile Lys Glu Ala Met
                245                 250                 255

Asp Arg Thr Phe Ala Lys Leu Glu Gln
            260                 265
```

**Beschreibung**

Die Erfindung bezieht sich auf ein Verfahren zum Identifizieren von Fungiziden, die Verwendung von Uridin-5'-Monophosphat-/Cytidin-5'-Monophosphat-Kinase zum Identifizieren von Fungiziden, auf Nukleinsäuren kodierend für UMP/CMP-Kinasen und die davon kodierten Polypeptide.

Unerwünschtes Pilzwachstum, das in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, welche eine entscheidende Bedeutung bei der Ausprägung der Pathogenität eines Pilzes haben.

Aufgabe der vorliegenden Erfindung war es deshalb, einen geeigneten neuen Angriffspunkt für potentielle fungizide Wirkstoffe zu identifizieren und zugänglich zu machen, und ein Verfahren zur Verfügung zu stellen, das die Identifizierung von Modulatoren dieses Angriffspunkts ermöglicht, die dann als Fungizide verwendet werden können.

Alle bekannten eukaryotischen Uridylat-Kinasen katalysieren sowohl die effiziente Phosphorylierung von UMP als auch von CMP zu den korrespondierenden Uridin- und Cytidin-Diphosphaten, wobei ATP als Phosphatdonor genutzt wird. Aus diesem Grund sind Uridylat-Kinasen (EC 2.7.4.-) auch als UMP/CMP-Kinasen (Uridin-5'-Monophosphat-/Cytidin-5'-Monophosphat-Kinasen) oder Pyrimidin-Nukleosid-Monophosphat-Kinasen bekannt (Yan und Tsai, 1999, Adv. Enzymol. Relat.

Areas Mol. Biol. 73:103-34). Die enzymatische Aktivität der UMP/CMP-Kinasen kann schematisch wie folgt dargestellt werden:

$$ATP + UMP \iff ADP + UDP, \text{ bzw. } ATP + (d)CMP \iff ADP + (d)CDP$$

Da alle Pyrimidine innerhalb der Zelle aus UMP abgeleitet werden, ist die UMP/CMP-Kinase ein Schlüsselenzym für die Pyrimidin-Nukleotid Biosynthese. Die von der UMP/CMP-Kinase katalysierte Reaktion ist ein essentieller Schritt für die Bereitstellung von UDP für weitere anabolische Stoffwechselwege, wie beispielsweise die RNA-Biosynthese oder die Synthese von Zuckernukleotiden wie UDP-Glukose und UDP-Galaktose (Ma et al., 1990, Journal of Biol. Chem. 256 (31): 19122-19127; Jong et al., 1993, Arch. Biochem. Biophys. 304 (1): 197-204; Zhou et al., 1998, Plant Physiol. 117: 245-254).

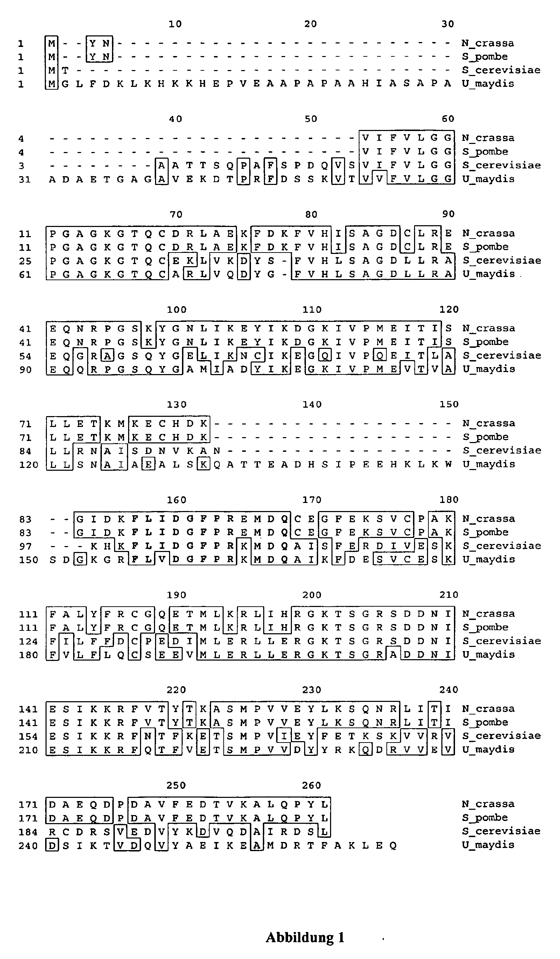

Gene für die UMP/CMP-Kinase wurden aus verschiedenen Pilzen kloniert. Aus den Hefen *Saccharomyces cerevisiae* (Swissprot Accession No.: P15700) und *Schizosaccharomyces pombe* (Swissprot Accession No.: 059771), aus *Lentinus edodes* (Pir Accession No.: JC6572) sowie dem Schleimpilz *Dictyostelium discoideum* (Swissprot Accession No.: P20425). Daneben wurde die UMP/CMP-Kinase auch aus anderen Organismen isoliert, so zum Beispiel aus *Homo sapiens* (Swissprot Accession No.: P30085), *Mus musculus* (Swissprot Accession No.: Q9DBP5), *Sus scrofa* (Swissprot Accession No.: Q29561), *Caenorhabditis elegans* (RefSeq Accession No.: NP_496386), *Arabidopsis thaliana* (Swissprot Accession No.: 004905) und *Oryza sativa* (Genbank Accession No.: AAF23371). Die Sequenzähnlichkeiten sind innerhalb der eukaryontischen Klassen signifikant (siehe Abbildung 1).

UMP/CMP-Kinasen wurden bislang z.B. aus *Saccharomyces cerevisiae, Arabidopsis thaliana* und *Lentinus edodes* isoliert, exprimiert, gereinigt und charakterisiert (Ma et al., 1990, Journal of Biol. Chem. 256 (31): 19122-19127; Zhou et al., 1998, Plant Physiol. 117: 245-254; Kaneko et al., 1998, Gene 211: 259-266).

Aufgabe der vorliegenden Erfindung war es nun, neue Angriffspunkte von Fungiziden in Pilzen, insbesondere in phytopathogenen Pilzen, zu identifizieren und ein Verfahren zugänglich zu machen, in denen Inhibitoren eines solchen Angriffspunktes bzw. Polypeptids identifiziert und auf ihre funigiziden Eigenschaften hin geprüft werden können. Die Aufgabe wurde gelöst, indem aus einem phytopathogenen Pilz, *Ustilago maydis,* die für eine UMP/CMP-Kinase kodierende Nukleinsäure isoliert, das davon kodierte Polypeptid gewonnen und ein Verfahren zur Verfügung gestellt wurde, mit dem Inhibitoren dieses Enzyms bestimmt werden können. Die mit diesem Verfahren identifizierten Inhibitoren können *in vivo* gegen Pilze eingesetzt werden.

**Beschreibung der Abbildungen**

**Abbildung 1:** Sequenz-Alignment pilzlicher UMP/CMP-Kinasen. Homologie zwischen UMP/CMP-Kinasen aus verschiedenen Pilzen. Rahmen geben Bereiche mit einer genau übereinstimmenden Sequenz wieder (Konsensussequenz). In der Abbildung sind weiterhin die dem Prosite Motiv für die ATPase Domäne von UMP/CMP Kinasen entsprechenden Aminosäuren gekennzeichnet. S_pombe: *Saccharomyces pombe.* N_crassa: *Neurospora crassa.* S_cerevisiae: *Saccharomyces cerevisiae.* U_maydis: *Ustilago maydis.*

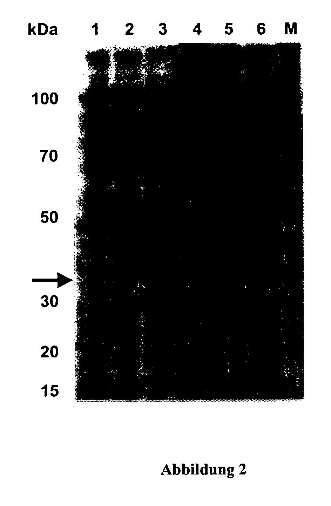

**Abbildung 2:** SDS-Gelchromatojzraphie. Gezeigt werden die Ergebnisse der heterologen Expression der UMP/CMP-Kinase in *E. coli* BL21(DE3)pLysS. Das überexprimierte His-Fusionsprotein hat eine Größe von ca. 33 kDa. In Spur "M" wurde ein Größenstandard aufgetragen. Spur 1: Membranfraktion nach Zellaufschluss und Trennung von Membran- und Cytoplasmafraktion mittels Zentrifugation, gewonnen aus Zellen vor Induktion mit IPTG. Spur 2: Membranfraktion, 3,5 h nach der Induktion mit 0,05 mM IPTG, Zellaufschluss und Trennung von Membran- und Cytoplasmafraktion mittels Zentrifugation. Spur 3: Membranfraktion 3,5 h nach der Induktion mit 0,5 mM IPTG, Zellaufschluss und Trennung von Membran- und Cytoplasmafraktion mittels Zentrifugation. Spur 4: Cytoplasmafraktion nach Zellaufschluss und Trennung von Membran- und Cytoplasmafraktion mittels Zentrifugation, gewonnen aus Zellen vor Induktion mit IPTG. Spur 5: Cytoplasmafraktion der überexprimierten UMP/CMP-Kinase 3,5 h Stunden nach der Induktion mit 0,05 mM IPTG, Zellaufschluss und Trennung von Membran- und Cytoplasmafraktion mittels Zentrifugation. Spur 6: Cytoplasmafraktion der überexprimierten UMP/CMP-Kinase 3,5 h Stunden nach der Induktion mit 0,5 mM IPTG, Zellaufschluss und Trennung von Membran- und Cytoplasmafraktion mittels Zentrifugation.

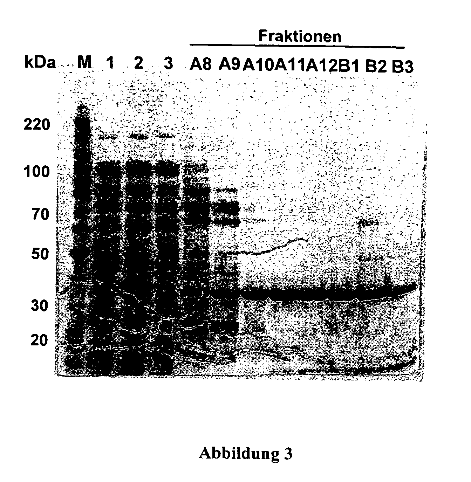

**Abbildung 3:** SDS-Gelchromatographie. Gezeigt werden die Ergebnisse der Reinigung der UMP/CMP-Kinase aus *U. maydis* mittels Affinitätschromatographie. Das überexprimierte His-Fusionsprotein hat eine Grösse von ca. 33 kDa. In Spur "M" wurde ein Größenstandard aufgetragen. Spur 1: Cytoplasmafraktion der überexprimierten UMP/CMP-Kinase 3,5 h Stunden nach der Induktion mit 0,5 mM IPTG, Zellaufschluss und Trennung von Membran- und Cytoplasmafraktion mittels Zentrifugation. Spur 2: Durchfluss zu Beginn der Auftragung der Cytoplasmafraktion auf die Affinitätschromatographiesäule. Spur 3: Durchfluss am Ende der Auftragung der Cytoplasmafraktion auf die Affinitätschromatographiesäule. Spuren A8 bis B3: Elutionsfraktionen mit gereinigter UMP/CMP-Kinase.

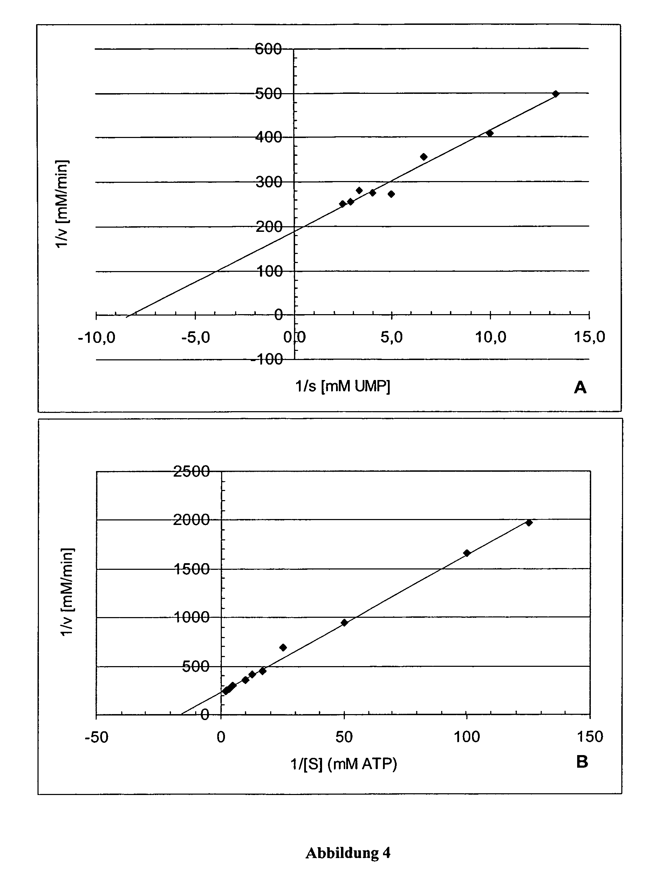

**Abbildung 4:** Graphische Darstellung der $K_M$-Wert Bestimmung für UMP (**A**) und ATP (**B**). Darstellung der gemessenen Werte nach Lineweaver und Burk: $1/V_o = 1/V_{max} + 1/S \times (K_M/V_{max})$, worin $V_o$ die anfängliche Reaktionsgeschwindigkeit, $V_{max}$ die maximal erreichbare Umsatzgeschwindigkeit und S die Substratkonzentration ist. $V_{max}$ und $K_M$ lassen sich dann als Abszissen- und Ordinatenabschnitt $1/V_{max}$ bzw. $1/K_M$ ablesen. Der $K_M$-Wert für UMP beträgt 0,12 mM, für ATP 0,06 mM.

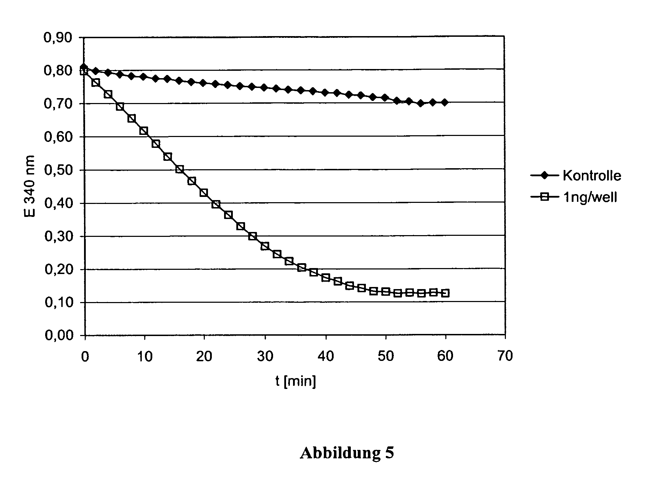

**Abbildung 5:** Reaktionskinetik der UMP/CMP Kinase aus *Ustilago maydis.* Der zeitabhängige Umsatz von ATP und UMP durch die UMP/CMP Kinase aus Ustilago maydis ist dargestellt. Das in der Reaktion entstehende ADP wird durch die gekoppelte Reaktion mit Pyruvatkinase und Lactatdehydrogenase gemessen. Dabei wird das in der Reaktion der Pyruvatkinase entstehende Pyruvat von der Lactatdehydrogenase unter Verbrauch von NADH zu Lactat umgesetzt. Der Nachweis des in der Gesamtreaktion umgesetzten NADH durch Absorptionsmessung bei 340 nm gibt daher Aufschluss über die enzymatische Aktivität der UMP/CMP Kinase. Die Kontrolle erfolgte durch Messung der Reaktion in Abwesenheit der UMP/CMP Kinase.

**SEQ ID NO:1** Nukleinsäuresequenz kodierend für die UMP/CMP-Kinase aus *Ustilago maydis.*

**SEQ ID NO: 2** Aminosäuresequenz der UMP/CMP-Kinase aus *Ustilago maydis.*

**Definitionen**

Unter dem Begriff "Homologie" bzw. "Identität" soll die Anzahl der übereinstimmenden Aminosäuren (Identität) mit anderen Proteinen, ausgedrückt in Prozent verstanden werden. Bevorzugt wird die Identität durch Vergleiche einer gegebenen Sequenz zu anderen Proteinen mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Die Identität kann standardmäßig mittels bekannten und der Öffentlichkeit zur Verfügung stehenden Computerprogrammen wie z.B. ClustalW (Thompson et al., *Nucleic Acids Research 22* (1994), 4673-4680) ermittelt werden. ClustalW wird z.B. öffentich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Intemetseiten, u.a. beim IGBMC (Institut de Genetique et de Biologie Moleculaire et Cellulaire, B.P.163, 67404 Illkirch Cedex, France; ftp://ftp-igbmc.u-strasbg.n/pub/) und beim EBI (ftp://ftp.ebi.ac.uk/pub/software/) sowie bei allen gespiegelten Intemetseiten des EBI (European Bioinformatics Institute, Wellcome Trust Genome Campus, Hinxton, Cambridge CB10 1SD, UK), heruntergeladen werden. Wenn das ClustalW Computerprogramm der Version 1.8 benutzt wird, um die Identität zwischen z.B. einem gegebenen Referenzprotein und anderen Proteinen zu bestimmen, sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP. Eine Möglichkeit zum Auffinden von ähnlichen Sequenzen ist die Durchführung von Sequenzdatenbankrecherchen. Hierbei wird eine oder werden mehrere Sequenzen als sogenannte Abfrage ("query") vorgegeben. Diese Abfragesequenz wird dann mittels statistischen Computerprogrammen mit Sequenzen, die in den ausgewählten Datenbanken enthalten sind, verglichen. Solche Datenbankabfragen ("blast searches") sind dem Fachmann bekannt und können bei verschiedenen Anbietern durchgeführt werden. Wird eine solche Datenbankabfrage z.B. beim NCBI (National Center for Biotechnology Information, http://www.ncbi.nlm.nih.gov/) durchgeführt, so sollen die Standardeinstellungen, die für die jeweilige Vergleichsanfrage vorgegeben sind, benutzt werden. Für Proteinsequenzvergleiche ("blastp") sind dieses folgende Einstellungen: Limit entrez = nicht aktiviert; Filter = low complexity aktiviert; Expect value = 10; word size = 3; Matrix = BLOSUM62; Gap costs: Existence = 11, Extension = 1. Als Ergebnis einer solchen Abfrage werden neben anderen Parametern auch der Anteil an Identität zwischen der Abfragesequenz und den in den Datenbanken aufgefundenen ähnlichen Sequenzen dargestellt. Unter einem erfindungsgemäßen Protein sollen daher im Zusammenhang mit der vorliegenden Erfindung solche Proteine verstanden werden, die bei der Verwendung mindestens einer der vorstehend beschriebenen Methoden zur Identitätsbestimmung eine Identität von mindestens 70 % aufweisen, bevorzugt von mindestens 75 %, besonders bevorzugt von mindestens 80 %, weiter bevorzugt von mindestens 85 %, und insbesondere von mindestens 90 %.

Der Ausdruck "hybridisieren" oder "Hybridisierung", wie er hierin verwendet wird, beschreibt den Vorgang, bei welchem ein einzelsträngiges Nukleinsäuremolekül mit einem komplementären Strang eine Basenpaarung eingeht. Auf diese Weise können z.B. ausgehend von der hierin genannten oder ableitbaren Sequenzinformation beispielsweise DNA-Fragmente aus anderen phytopathogenen Pilzen als *Ustilago maydis* isoliert werden, welche für UMP/CMP-Kinasen kodieren, welche dieselben oder ähnliche Eigenschaften einer der erfindungsgemäßen UMP/CMP-Kinase aufweisen. Zur Hybridisierung werden bevorzugt kurze Oligonukleotide mit einer Länge von etwa 10-50 bp, vorzugsweise von 15-40 bp beispielsweise der konservierten oder sonstigen Bereiche, die über Vergleiche mit anderen verwandten Genen in dem Fachmann bekannter Weise ermittelt werden können, verwendet. Es können aber auch längere Fragmente der erfindungsgemäßen Nukleinsäuren oder die vollständigen Sequenzen für die Hybridisierung verwendet werden. Unter Standardbedingungen sind beispielsweise je nach Nukleinsäure Temperaturen zwischen 42 und 58°C in einer wässrigen Pufferlösung mit einer Konzentration von 0,1 bis 5 x SSC oder zusätzlich die Gegenwart von 50 % Formamid zu verstehen, beispielsweise 42°C in 5 x SSC, 50 % Formamid. Vorteilhafterweise liegen die Hybridisierungsbedingungen für DNA:DNA-Hybride bei 0,1 x SSC und Temperaturen zwischen etwa 20°C bis 45°C, bevorzugt zwischen etwa 30°C bis 45°C. Für DNA: RNA-Hybride liegen die Hybridisierungsbedingungen bevorzugt bei 0,1 x SSC und Temperaturen zwischen etwa 30°C und 55°C, bevorzugt zwischen etwa 42°C und 55°C. Diese Temperaturen für die Hybridisierung sind beispielhafte Schmelztemperaturwerte für eine Nukleinsäure mit einer Länge von etwa 100 Nukleotiden und einem G + C-Gehalt von 50 % bei Abwesenheit von Formamid. Die experimentellen Bedingungen für die DNA- Hybridisierung sind in einschlägigen Lehrbüchern wie beispielsweise "Sambrook et al., "Molecular Cloning", Cold Spring Harbor Laboratory, 1989" beschrieben und lassen sich nach dem Fachmann bekannten Formeln beispielsweise abhängig von der Länge der Nukleinsäuren, der Art der Hybride oder dem G + C-Gehalt berechnen. Hybridisierungsbedingungen werden nach folgender Formel näherungsweise berechnet: Schmelztemperatur $Tm = 81,5°C + 16,6 \{log[c(Na^+)]\} + 0,41(\% \, G + C) - (500/n)$ (Lottspeich, F., Zorbas H. (Hrsg.). (1998). Bioanalytik. Spektrum Akademischer Verlag, Heidelberg, Berlin). Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM Na$^+$ (entspricht 0.1 x SSC) gewaschen. 1 x SSC-Puffer setzt sich wie folgt zusammen: 0,15 M NaCl, 15 mM Natriumcitrat, pH 7,2. Bevorzugte Hybridisierungsbedingungen sind nachstehend angegeben: Hybridisierungslösung: DIG Easy Hyb (Roche, ZZ) Hybridisierungstemperatur: 40°C bis 70°C, bevorzugt bei 42-55°C (DNA-DNA) oder 50°C (DNA-RNA). Dabei kann z.B. ein erster Waschschritt mit 2 x SSC, 0,1 % SDS 2 x 5 min bei Raumtemperatur erfolgen, gefolgt von einem zweiten Waschschritt mit 1

Der Begriff " UMP/CMP-Kinase Hemmtest"

x SSC, 0,1 % SDS 2 x 15 min bei 50°C.

Der Ausdruck "vollständige UMP/CMP-Kinase", wie er hierin verwendet wird, beschreibt eine UMP/CMP-Kinase, die von einer vollständigen kodierenden Region einer Transkriptionseinheit kodiert wird, beginnend mit dem ATG-Startcodon und umfassend alle informationstragenden Exonbereiche des im Herkunftsorganismus vorliegenden, für UMP/CMP-Kinase kodierenden Gens, sowie die für eine korrekte Termination der Transkription nötigen Signale.

Der Ausdruck "biologische Aktivität einer UMP/CMP-Kinase", wie er hierin verwendet wird, bezieht sich auf die Fähigkeit eines Polypeptids, die vorstehend beschriebene Reaktion zu katalysieren, d.h. die Umsetzung von Uridinmonophosphat und Cytidinmonophosphat zu Uridindiphosphat und Cytidindiphosphat.

Der Ausdruck "aktives Fragment", wie er hierin verwendet wird, beschreibt nicht mehr vollständige Nukleinsäuren kodierend für UMP/CMP-Kinasen, die aber noch für Polypeptide mit der biologischen Aktivität einer UMP/CMP-Kinase kodieren, und die eine für die UMP/CMP-Kinase charakteristische Reaktion wie vorne beschrieben katalysieren können. Solche Fragmente sind kürzer als die oben beschriebenen vollständigen, für die UMP/CMP-Kinase kodierenden Nukleinsäuren. Dabei können sowohl an den 3'- und/oder 5'-Enden der Sequenz Nukleinsäuren entfernt worden sein, es können aber auch Teile der Sequenz deletiert, d.h. entfernt worden sein, die die biologische Aktivität der UMP/CMP-Kinase nicht entscheidend beeinträchtigen. Eine geringere oder gegebenenfalls auch eine erhöhte Aktivität, die aber noch die Charakterisierung bzw. Verwendung des resultierenden UMP/CMP-Kinase Fragments gestattet, wird dabei als ausreichend im Sinne des hier verwendeten Ausdrucks verstanden. Der Ausdruck "aktives Fragment" kann sich ebenso auf die Aminosäuresequenz der UMP/CMP-Kinase beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist. Die Fragmente können dabei verschiedene Längen besitzen.

Der Begriff " UMP/CMP-Kinase Hemmtest" oder "Hemmtest", wie er hierin verwendet wird, bezieht sich auf ein Verfahren bzw. einen Test, der es gestattet, die Inhibition der enzymatischen Aktivität eines Polypeptids mit der Aktivität einer UMP/CMP-Kinase durch eine oder mehrere chemische Verbindungen (Kandidaten- oder Testverbindung (en)) zu erkennen, wodurch die chemische Verbindung als Inhibitor der UMP/CMP-Kinase identifiziert werden kann.

Der Ausdruck "Gen", wie er hierin verwendet wird, ist die Bezeichnung für einen Abschnitt aus dem Genom einer Zelle, der für die Synthese einer Polypeptid-Kette verantwortlich ist.

Der Ausdruck "Fungizid" bzw. "fungizid", wie er hierin verwendet wird, bezieht sich auf chemische Verbindungen, die zur Bekämpfung von Pilzen, insbesondere von pflanzenpathogenen Pilzen geeignet sind. Solche pflanzenpathogenen Pilze werden nachfolgend genannt, wobei die Aufzählung nicht abschließend ist:

Plasmodiophoromycetes, Oomycetes, Chytridiomycetes, Zygomycetes, Ascomycetes, Basidiomycetes und Deuteromycetes, z.B.

Pythium-Arten, wie beispielsweise *Pythium ultimum,* Phytophthora-Arten, wie beispielsweise *Phytophthora infestans,* Pseudoperonospora-Arten, wie beispielsweise *Pseudoperonospora humuli* oder *Pseudoperonospora cubensis,* Plasmopara-Arten, wie beispielsweise *Plasmopara viticola,* Bremia-Arten, wie beispielsweise *Bremia lactucae,* Peronospora-Arten, wie beispielsweise *Peronospora pisi* oder *P. brassicae,* Erysiphe-Arten, wie beispielsweise *Erysiphe graminis,* Sphaerotheca-Arten, wie beispielsweise *Sphaerotheca fuliginea,* Podosphaera-Arten, wie beispielsweise *Podosphaera leucotricha,* Venturia-Arten, wie beispielsweise *Venturia inaequalis,* Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita,* Sclerotinia-Arten, wie beispielsweise *Sclerotinia sclerotiorum,* Tilletia-Arten, wie beispielsweise *Tilletia caries;* Ustilago-Arten, wie beispielsweise *Ustilago nuda* oder *Ustilago avenae,* Pellicularia-Arten, wie beispielsweise *Pellicularia sasakii,* Pyricularia-Arten, wie beispielsweise *Pyricularia oryzae,* Fusarium-Arten, wie beispielsweise *Fusarium culmorum,* Botrytis-Arten, Septoria-Arten, wie beispielsweise *Septoria nodorum,* Leptosphaeria-Arten, wie beispielsweise *Leptosphaeria nodorum,* Cercospora-Arten, wie beispielsweise *Cercospora canescens,* Altemaria-Arten, wie beispielsweise *Alternaria brassicae* oder Pseudocercosporella-Arten, wie beispielsweise *Pseudocercosporella herpotrichoides.*

Von besonderem Interesse sind z.B. auch *Magnaporthe grisea, Cochliobulus heterostrophus, Nectria hematococcus* and Phytophtora species.

Fungizide Wirkstoffe, die mit Hilfe der erfindungsgemäßen UMP/CMP-Kinase gefunden werden, können aber auch mit UMP/CMP-Kinase aus humanpathogenen Pilzspezies interagieren, wobei die Interaktion mit den unterschiedlichen in diesen Pilzen vorkommenden UMP/CMP-Kinasen nicht immer gleich stark sein muss.

Gegenstand der vorliegenden Erfindungen ist deshalb auch ein Verfahren zum Identifizieren von Antimykotika, d.h. von Inhibitoren der UMP/CMP-Kinase human- oder tierpathogener Pilze, die zur Herstellung von Mitteln zur Behandlung von durch human- oder tierpathogene Pilze hervorgerufenen Erkrankungen verwendet werden können.

Dabei sind die folgenden humanpathogenen Pilze von besonderem Interesse, die unter anderem die nachfolgend genannten Krankheitsbilder hervorrufen können:

Dermatophyten, wie z.B. Trichophyton spec., Microsporum spec., *Epidermophyton floccosum* oder *Keratomyces ajelloi,* die z.B. Fußmykosen (Tinea pedis) hervorrufen,

Hefen, wie z.B. *Candida albicans,* die Soor-Ösophagitis und Dermatitis hervorruft, *Candida glabrata, Candida krusei* oder *Cryptococcus neoformans,* die z.B. pulmonale Cryptococcose und auch Torulose hervorrufen können,

Schimmelpilze, wie z.B. *Aspergillus fumigatus, A. flavus, A. niger,* die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi,* der z.B. chronische granulomatöse Pharyngitis und Tracheitis hervorruft, *Madurella myzetomatis,* der z.B. subkutane Myzetome hervorruft, *Histoplasma capsulatum,* der z.B. retikuloendotheliale Zytomykose und M. Darling hervorruft, *Coccidioides immitis,* der z. B. pulmonale Coccidioidomykose und Sepsis hervorruft, *Paracoccidioides brasiliensis,* der z.B. brasilianische Blastomykose hervorruft, *Blastomyces dermatitidis,* der z.B. Gilchrist-Krankheit und nordamerikanische Blastomykose hervorruft, *Loboa loboi,* der z.B. Keloid-Blastomykose und Lobo's Krankheit hervorruft, und *Sporothrix schenckii,* der z.B. Sporotrichose (granulomatöse Hautmykose) hervorruft.

Im Folgenden sollen die Begriffe "fungizid" bzw. "Fungizid" gleichermaßen für die Begriffe "antimykotisch" bzw. "Antimykotikum" als auch für die Begriffe "fungizid" bzw. "Fungizid" im herkömmlichen Sinne, d.h. bezogen auf pflanzenpathogene Pilze, verwendet werden.

Fungizide Wirkstoffe, die mit Hilfe einer aus einem bestimmten Pilz, hier z.B. aus *Ustilago maydis,* gewonnenen UMP/CMP-Kinase gefunden werden, können auch mit einer UMP/CMP-Kinase aus zahlreichen anderen Pilzspezies, gerade auch aus pflanzenpathogenen Pilzen interagieren, wobei die Interaktion mit den unterschiedlichen in diesen Pilzen vorkommenden UMP/CMP-Kinasen nicht immer gleich stark sein muss. Dies erklärt unter anderem die beobachtete Selektivität der an diesem Enzym wirksamen Substanzen.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Der Ausdruck "Kompetitor", wie er hierin verwendet wird, bezieht sich auf die Eigenschaft der Verbindungen, mit anderen, gegebenenfalls zu identifizierenden Verbindungen um die Bindung an der UMP/CMP-Kinase zu kompetitieren und diese vom Enzym zu verdrängen bzw. von dieser verdrängt zu werden.

Der Ausdruck "Agonist", wie er hierin verwendet wird, bezieht sich auf ein Molekül, das die Aktivität der UMP/CMP-Kinase beschleunigt oder verstärkt.

Der Ausdruck "Antagonist", wie er hierin verwendet wird, bezieht sich auf ein Molekül, das die Aktivität der UMP/CMP-Kinase verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden bzw. die deren Aktivität beeinflussen. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Ligand en darstellen oder strukturelle oder funktionelle Mimetika davon. Bevorzugt handelt es sich beim Ausdruck "Modulator", wie er hierin verwendet wird, jedoch um solche Moleküle, die nicht die natürlichen Substrate bzw. Liganden darstellen.

Der Ausdruck "Inhibitor" bzw. "spezifischer Inhibitor", wie er hierin gebraucht wird, bezeichnet eine Substanz, in deren Gegenwart konzentrationsabhängig eine signifikante Abnahme der enzymatischen Aktivität der UMP/CMP Kinase erfolgt. Ein solcher Inhibitor ist vorzugsweise "spezifisch", d.h. er beeinflußt die Enzymaktivität der UMP/CMP Kinase negativ durch gezielte Bindung an definierte (katalytische oder regulatorische) Zentren. Dabei ist die Konzentration des spezifischen Inhibitors niedriger als die Konzentration eines Inhibitors, der das Enzym unspezifisch hemmt. Vorzugsweise ist die Konzentration zweifach niedriger, insbesondere bevorzugt fünffach niedriger und ganz besonders bevorzugt zumindest zehnfach oder 20fach niedriger als die Konzentration einer Verbindung, die zum Hervorrufen eines unspezifischen Effekts benötigt wird.

## Beschreibung der Erfindung

Trotz umfangreicher Forschungen an der UMP/CMP-Kinase war bislang unbekannt, dass die UMP/CMP-Kinase in Pilzen ein Zielprotein (ein sogenanntes "Target") fungizid wirksamer Substanzen sein kann. Damit wird in der vorliegenden Erfindung zum ersten Mal gezeigt, dass die UMP/CMP-Kinase ein insbesondere für Pilze wichtiges Enzym darstellt und deshalb in besonderem Maße dazu geeignet ist, als Zielprotein für die Suche nach weiteren und verbesserten fungizid wirksamen Wirkstoffen verwendet zu werden.

Im Rahmen der vorliegenden Erfindung wurde mit Hilfe von Knock-Out Experimenten gezeigt (Beispiel 1), dass die UMP/CMP-Kinase ein essentielles Enzym für Pilze ist und deshalb auch ein Angriffspunkt oder "Target" für fungizide Wirkstoffe sein kann. Damit wird in der vorliegenden Erfindung zum ersten Mal gezeigt, dass die UMP/CMP-Kinase ein insbesondere für Pilze wichtiges Enzym darstellt und deshalb in besonderem Maße dazu geeignet ist, als Zielprotein für die Suche nach weiteren und verbesserten fungizid wirksamen Wirkstoffen verwendet zu werden.

Im Rahmen der vorliegenden Erfindung wurde weiterhin ein Verfahren entwickelt, das geeignet ist, die Aktivität der UMP/CMP-Kinase sowie die Hemmung dieser Aktivität in einem sogenannten Hemmtest zu bestimmen und auf diese Weise Inhibitoren des Enzyms z.B. in HTS- und UHTS-Verfahren zu identifizieren und deren fungizide Eigenschaften zu prüfen.

Im Rahmen der vorliegenden Erfindung wurde so gefunden, dass die UMP/CMP-Kinasen auch *in vivo* durch Wirkstoffe inhibiert werden können und ein mit diesen Wirkstoffen behandelter pilzlicher Organismus durch die Behandlung mit diesen Wirkstoffen geschädigt bzw. abgetötet werden kann. Die Inhibitoren einer pilzlichen UMP/CMP-Kinase können also als Fungizide insbesondere im Pflanzenschutz oder auch als Antimykotika in Pharmaindikationen verwendet werden. Im Rahmen der vorliegenden Erfindung kann gezeigt werden, dass die Hemmung der UMP/CMP-Kinase mit einer in einem erfindungsgemäßen Verfahren identifizierten Substanzen zum Absterben oder zu einer Wachstumshemmung der behandelten Pilze in synthetischen Medien bzw. auf der Pflanze führt.

UMP/CMP-Kinasen können aus verschiedenen pflanzenpathogenen oder auch aus human- oder tierpathogenen Pilzen gewonnen werden, z.B. wie in der vorliegenden Erfindung gezeigt aus dem pflanzenpathogenen Pilz *U. maydis.* Zur Herstellung der UMP/CMP-Kinasen aus Pilzen kann das Gen z.B. rekombinant in *Escherichia coli* exprimiert und aus *E. coli* Zellen eine Enzympräparation hergestellt werden. Bevorzugt werden UMP/CMP-Kinasen aus pflanzenpathogenen Pilzen verwendet, um im Pflanzenschutz einsetzbare Fungizide zu identifizieren. Ist das Ziel die Identifizierung von Fungiziden bzw. Antimycotika, die in Pharmaindikationen verwendet werden sollen, empfiehlt sich der Einsatz von UMP/CMP-Kinasen aus human- bzw. tierpathogenen Pilzen.

So wurde für die Expression des von *uck* kodierten Polypeptids UCK der zugehörige ORF mittels RT-PCR aus Gesamt-RNA nach dem Fachmann bekannten Methoden über genspezifische Primer amplifiziert. Die entsprechende DNA wurde in den Vektor pDEST17 (Gateway Vektor der Firma Invitrogen, versehen mit einem N-terminalen HIS-Tag) kloniert. Das resultierende Plasmid pDEST17-UCK enthält die vollständige kodierende Sequenz von *uck* in einer N-terminalen Fusion mit einem HIS-Tag aus dem Vektor. Das UCK Fusionsprotein besitzt eine berechnete Masse von 33 kDa (Beispiel 2 und Abbildung 2).

Das Plasmid pDEST17-UCK wurde dann zur rekombinanten Expression von UCK in *E. coli* BL21 (DE3) pLysS verwendet (Beispiel 2).

Wie bereits vorstehend ausgeführt, ist die vorliegende Erfindung nicht nur auf die Verwendung von UMP/CMP-Kinase aus *Ustilago maydis* beschränkt. In analoger und dem Fachmann bekannter Weise können auch aus anderen Pilzen, vorzugsweise aus pflanzenpathogenen Pilzen, Polypeptide mit der Aktivität einer UMP/CMP-Kinase gewonnen werden, die dann in einem erfindungsgemäßen Verfahren eingesetzt werden können. Bevorzugt wird die UMP/CMP-Kinase aus *Ustilago maydis* verwendet.

UMP/CMP-Kinasen teilen sich homologe Bereiche (siehe Abbildung 1 ). Typisch für UMP/CMP-Kinase ist ein konservierter Bereich, der üblicherweise in ATP-bindenden Enzymen gefunden werden kann. Dieser Bereich umfasst einen Asparaginsäure-Rest, der mit der katalytischen Aktivität der UMP/CMP-Kinasen in Verbindung gebracht wird und an einer Salzbrücke beteiligt sein dürfte. Weiterhin wird auch ein Arginin-Rest umfasst, dessen Modifikation die Aktivität des Enzyms beeinträchtigt.

Diese beiden Aminosäurereste sind ebenso wie ihre Sequenzumgebung ein für UMP/CMP-Kinasen charakteristisches Sequenzmerkmal. Dieses für UMP/CMP-Kinasen typische Sequenzmotiv kann in Form eines Prosite-Motivs wiedergegeben werden (Hofmann K., Bucher P., Falquet L., Bairoch A. (1999) "The PROSITE database, its status in 1999". *Nucleic Acids Res.* 27, 215). Es kann wie folgt dargestellt werden:

$$\text{[LIVMFYWCA]-[LIVMFYW](2)-D-G-[FYI]-P-R-x(3)-[NQ],}$$

wobei der Rest D für Asparaginsäure im aktiven Zentrum steht und der Rest G für Arginin.

Vorzugsweise werden UMP/CMP-Kinasen mit dem Sequenzmotiv

F-L-[IV]-D-G-F-P-R-x(3)-Q

verwendet.

PROSITE ermöglicht Polypeptiden eine Funktion zuzuordnen und somit UMP/CMP-Kinasen als solche zu erkennen. Bei der Darstellung des Prosite Motivs wird der "Ein-Buchstaben-Code" verwendet. Das Symbol "x" steht für eine Position, an der jede Aminosäure akzeptiert wird. Eine variable Position, an der verschiedene bestimmte Aminosäuren akzeptiert werden, wird in eckigen Klammern "[...]" dargestellt, wobei die an dieser Position möglichen Aminosäuren aufgezählt werden. Aminosäuren, die an einer bestimmten Position nicht akzeptiert werden, stehen dagegen in geschweiften Klammern "{...}". Ein Gedankenstrich "-" trennt die einzelnen Elemente bzw. Positionen des Motivs. Wiederholt sich eine bestimmte Position, z.B. "x", mehrfach hintereinander, kann dies durch Angabe der Zahl der Wiederholungen in einer nachfolgenden Klammer dargestellt werden, z.B. "x (3)", was für "x-x-x" steht.

Ein Prosite Motiv stellt also letztlich die Komponenten einer Konsensussequenz dar sowie Abstände zwischen den beteiligten Aminosäuren und ist damit typisch für eine bestimmte Enzymklasse. Anhand dieses Motivs können auf Basis der erfindungsgemäßen Nukleinsäuren weitere Polypeptide aus pflanzenpathogenen Pilzen indentifiziert bzw. zugeordnet werden, die zur selben Klasse wie das erfindungsgemäße Polypeptid gehören und deshalb auch in erfindungsgemäßer Weise verwendet werden können.

Im Falle der UMP/CMP-Kinase aus *U. maydis* liegt dieses Motiv ebenso vor wie bei *S. cerevisiae, S. pombe,* oder *N. crassa* (vgl. Abbildung 1).

Das oben genannte Prosite Motiv bzw. die spezifische Konsensussequenz sind typisch für die erfindungsgemäßen Polypeptide, die anhand dieser Konsensussequenzen strukturell definiert werden können und damit auch eindeutig identifizierbar sind.

Gegenstand der vorliegenden Erfindung sind deshalb auch Polypeptide aus pflanzenpathogenen Pilzen mit der biologischen Aktivität einer UMP/CMP-Kinase, die das vorstehend genannte Prosite Motiv [LIVMFYWCA]-[LIVMFYW](2)-D-G-[FYI]-P-R-x(3)-[NQ], besonders bevorzugt das Motiv F-L-[IV]-D-G-F-P-R-x(3)-Q umfassen.

Aufgrund der Homologien, die bei speziesspezifischen Nukleinsäuren kodierend für UMP/CMP-Kinasen vorliegen, können auch UMP/CMP-Kinasen aus anderen pflanzenpathogenen Pilzen identifiziert und verwendet werden, um die oben gestellte Aufgabe zu lösen, d.h. sie können ebenfalls zum Identifizieren von Inhibitoren einer UMP/CMP-Kinase verwendet werden, welche wiederum als Fungizide im Pflanzenschutz verwendet werden können. Es ist jedoch auch denkbar einen anderen Pilz, der nicht pflanzenpathogen ist, bzw. dessen UMP/CMP-Kinase oder die dafür kodierende Sequenz zu verwenden, um fungizid wirkende Inhibitoren der UMP/CMP-Kinase zu identifizieren. Aufgrund der hier angegebenen Sequenz gemäß SEQ ID NO: 1 und eventuell davon abgeleiteten Primern sowie gegebenenfalls unter Zuhilfenahme des vorstehend gezeigten Prosite Motivs ist es dem Fachmann möglich, z.B. mittels PCR weitere für UMP/CMP-Kinasen kodierende Nukleinsäuren aus anderen (pflanzenpathogenen) Pilzen zu erhalten und zu identifizieren. Solche Nukleinsäuren und deren Verwendung in Verfahren zum Identifizieren von fungiziden Wirkstoffen werden als von der vorliegenden Erfindung umfasst betrachtet.

Mit Hilfe der erfindungsgemäßen Nukleinsäuresequenz sowie gemäß den vorstehend beschriebenen Verfahren erhaltene Sequenzen aus anderen pflanzenpathogenen Pilzen können weitere für eine UMP/CMP-Kinase kodierende Nukleinsäuresequenzen aus anderen, insbesondere aus pflanzenpathogenen Pilzen identifiziert werden. Gegenstand der vorliegenden Erfindung sind daher Nukleinsäuren aus pflanzenpathogenen Pilzen, die für ein Polypeptid mit der enzymatischen Aktivität einer UMP/CMP-Kinase kodieren, insbesondere Polypeptide, die die vorstehend beschriebenen Motive umfassen.

Bevorzugt sind Gegenstand der vorliegenden Erfindung Nukleinsäuren aus den vorstehend unter Definitionen genannten pflanzenpathogenen Pilzspezies, die für ein Polypeptid mit der enzymatischen Aktivität einer UMP/CMP-Kinase kodieren.

Gegenstand der vorliegenden Erfindung ist insbesondere bevorzugt die für die UMP/CMP-Kinase aus *Ustilago maydis* kodierende Nukleinsäure mit der SEQ ID NO: 1 sowie die für die Polypeptide gemäß SEQ ID NO:2 oder aktive Fragmente davon kodierenden Nukleinsäuren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, und insbesondere cDNAs.

Besonders bevorzugt umfassen die erfindungsgemäßen Nukleinsäuren eine Sequenz aus pflanzenpathogenen Pilzen kodierend für ein Polypeptid mit der enzymatischen Aktivität einer UMP/CMP-Kinase ausgewählt aus

a) einer Sequenz gemäß SEQ ID NO: 1,

b) Sequenzen, die für ein Polypeptid kodieren, welches die Aminosäuresequenz gemäß SEQ ID NO: 2 umfasst,

c) Sequenzen, die für ein Polypeptid kodieren, welches das Motiv [LIVMFYWCA]-[LIVMFYW](2)-D-G-[FYI]-P-R-x(3)-[NQ], besonders bevorzugt das Motiv F-L-[IV]-D-G-F-P-R-x(3)-Q umfasst,

d) Sequenzen, welche an die unter a) und b) definierten Sequenzen bei einer Hybridisierungstemperatur von 42-65°C hybridisieren, und

e) Sequenzen, welche eine zumindest 80 %-ige, bevorzugt zumindest 85 %-ige und besonders bevorzugt eine zumindest 90 %-ige Identität mit den unter a) und b) definierten Sequenzen aufweisen.

Wie bereits vorstehend ausgeführt, ist die vorliegende Erfindung nicht nur auf die UMP/CMP-Kinase aus *Ustilago maydis* beschränkt. In analoger und dem Fachmann bekannter Weise können auch aus anderen Pilzen, vorzugsweise aus pflanzenpathogenen Pilzen, Polypeptide mit der Aktivität einer UMP/CMP-Kinase gewonnen werden, die dann z.B. in einem erfindungsgemäßen Verfahren eingesetzt werden können. Bevorzugt wird die UMP/CMP-Kinase aus *Ustilago maydis* verwendet.

Gegenstand der vorliegenden Erfindung sind weiterhin DNA-Konstrukte, die eine erfindungsgemäße Nukleinsäure und einen homologen oder heterologen Promotor umfassen.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promotor des Blumenkohlmosaikvirus für pflanzliche Zellen, der Promotor der Alkoholdehydrogenase für Hefezellen, die T3-, T7- oder SP6-Promotoren für prokaryotische Zellen oder zellfreie Systeme. Promotoren, die zur Expression der UMP/CMP-Kinasen in gramnegativen Bakterienstämmen ebenfalls geeignet sind, sind beispielsweise der cos-, tac-, trp-, tet-, lpp-, lac-, lacIq-, T5-, gal-, trc-, ara-, 1-PR- oder 1-PL-Promotor. Zur Expression in Hefestämmen können außerdem die Promotoren ADC1, MFa, AC, P-60, CYC1, GAPDH, TEF, rp28, AOX1 und GAP genutzt werden. Für Insektenzellen sind beispielsweise der Polyhedrin-Promotor sowie der p10-Promotor (Luckow, V. A. and Summers, M. D. (1988) Bio/Techn. 6, 47-55) einsetzbar.

Bevorzugt sollten pilzliche Expressionssysteme wie z.B. das *Pichia pastoris*-System verwendet werden, wobei hier die Transkription durch den Methanol induzierbaren AOX-Promotor angetrieben wird.

Gegenstand der vorliegenden Erfindung sind ferner Vektoren, die eine erfindungsgemäße Nukleinsäure, eine erfindungsgemäße regulatorische Region oder ein erfindungsgemäßes DNA-Konstrukt enthalten. Als Vektoren können alle in molekularbiologischen Laboratorien verwendete Phagen, Plasmide, Phagmide, Phasmide, Cosmide, YACs, BACs, künstliche Chromosomen oder Partikel, die für einen Partikelbeschuss geeignet sind, verwendet werden.

Bevorzugte Vektoren sind z.B. die kommerziell erhältlichen Fusions- und Expressionsvektoren pGEX (Pharmacia Biotech Inc), pMAL (New England Biolabs, Beverly, MA) and pRIT5 (Pharmacia, Piscataway, NJ) welches Gluthathion S-transferase beinhaltet (GST), Maltose Bindeprotein, oder Protein A, die pTrc-Vektoren (Amann et al., (1988), Gene 69:301-315), der "pKK233-2n (CLONTECH, Palo Alto, Californien) und die "pET"- und "pBADH-Vektor-Serien von Stratagene, La Jolla. Weitere vorteilhafte Vektoren zur Verwendung in Hefe sind pYep Secl (Baldari et al. (1987), Embo J. 6:229-234), pMFa (Kurjan and Herskowitz (1982), Cell 30:933-943), pJRY88 (Schultz et al. (1987), Gene 54: 113-123), and pYES-Derivate, pGAPZ-Derivate, pPICZ-Derivate, die Vektoren des Pichia Expression Kit' (Invitrogen Corporation, San Diego, CA), die p4XXprom. Vektorserie (Mumberg et al., 1995) sowie pSPORT-Vektoren (Fa. Life Technologies) für bakterielle Zellen, oder Gateway Vektoren (Fa. Life Technologies) für verschiedene Expressionssysteme in bakteriellen Zellen, Pflanzen, *P. pastoris, S. cerevisiae* oder Insektenzellen.

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten. Als Wirtszellen eignen sich sowohl prokaryotische Zellen, vorzugsweise *E. coli,* aber auch Bakterien der Gattungen Erwinia, Flavobacterium, Alcaligenes oder Cyanobakterien der Gattung Synechocystis oder Anabena, als auch Pilzzellen, wie die Hefen Saccharomyces, Candida oder Pichia, bevorzugt *Saccharomyces cerevisiae* oder *Pichia pastoris,* sowie die Pilze Aspergillus, Trichoderma, Ashbya, Neurospora, Fusarium, Beauveria, Mortierella oder Phythium, oder auch Insekten, Pflanzen, Froschoozyten und Zelllinien von Säugern.

Gegenstand der vorliegenden Erfindung sind weiterhin Polypeptide mit der biologischen Aktivität einer UMP/CMP-Kinase, die von den erfindungsgemäßen Nukleinsäuren kodiert werden.

Bevorzugt umfassen die erfindungsgemäßen Polypeptide eine Aminosäuresequenz aus pflanzenpathogenen Pilzen ausgewählt aus

(a) der Sequenz gemäß SEQ ID NO:2,

(b) Sequenzen, welche eine zumindest 80 %-ige, bevorzugt eine zumindest 85 %-ige, besonders bevorzugt eine 90 %-ige und insbesondere bevorzugt eine 95 %-ige Identität mit der unter a) definierten Sequenz haben,

(c) den unter b) angegebenen Sequenzen, welche das Motiv [LIVMFYWCA]-[LIVMFYW](2)-D-G-[FYI]-P-R-x(3)-[NQ], besonders bevorzugt das Motiv F-L-[IV]-D-G-F-P-R-x(3)-Q umfassen, und

(d) Fragmenten der unter a) bis c) angegebenen Sequenzen, welche die gleiche biologische Aktivität aufweisen wie die unter a) definierte Sequenz.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezemierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können. In den erfindungsgemäßen Verfahren können ebenso aktive Fragmente einer UMP/CMP-Kinase eingesetzt werden, solange sie die Bestimmung der enzymatischen Aktivität des Polypeptids bzw. deren Inhibition durch eine Kandidatenverbindung ermöglichen.

Die in den erfindungsgemäßen Verfahren eingesetzten Polypeptide können im Vergleich zu den entsprechenden Regionen von natürlich vorkommenden UMP/CMP-Kinasen Deletionen oder Aminosäuresubstitutionen aufweisen, solange sie zumindest noch die biologische Aktivität einer vollständigen UMP/CMP-Kinase zeigen. Konservative Substitutionen sind bevorzugt. Solche konservativen Substitutionen umfassen Variationen, wobei eine Aminosäure durch eine andere Aminosäure aus der folgenden Gruppe ersetzt wird:

1. Kleine aliphatische, nicht-polare oder wenig polare Reste: Ala, Ser, Thr, Pro und Gly;

2. Polare, negativ geladene Reste und deren Amide: Asp, Asn, Glu und Gln;

3. Polare, positiv geladene Reste: His, Arg und Lys;

4. Große aliphatische, nicht-polare Reste: Met, Leu, Ile, Val und Cys; und

5. Aromatische Reste: Phe, Tyr und Trp.

Ein mögliches Reinigungsverfahren der UMP/CMP-Kinase basiert auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie oder Affinitätschromatographie (vgl. Beispiel 2).

Ein schnelles Verfahren zum Isolieren von UMP/CMP-Kinasen, die von Wirtszellen synthetisiert werden, beginnt mit der Expression eines Fusionsproteins, wobei der Fusionspartner auf einfache Weise affinitätsgereinigt werden kann. Der Fusionspartner kann beispielsweise ein HIS-Tag sein (vgl. Beispiel 2). Das Fusionsprotein kann dann beispielsweise an einer $Ni^{2+}$-Säule gereinigt werden. Der Fusionspartner kann durch partielle proteolytische Spaltung beispielsweise an Linkem zwischen dem Fusionspartner und dem zu reinigenden erfindungsgemäßen Polypeptid abgetrennt werden. Der Linker kann so gestaltet werden, dass er Ziel-Aminosäuren wie Argininund Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z. B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Das Verfahren zum Herstellen von Polypeptiden mit der Aktivität einer UMP/CMP-Kinase, wie z.B. des Polypeptids *uck,* ist damit gekennzeichnet durch

(a) das Kultivieren einer Wirtszelle, enthaltend zumindest eine exprimierbare Nukleinsäuresequenz kodierend für ein Polypeptid aus Pilzen mit der biologischen Aktivität einer UMP/CMP-Kinase unter Bedingungen, die die Expression dieser Nukleinsäure gewährleisten, oder

(b) das Exprimieren einer exprimierbaren Nukleinsäuresequenz, kodierend für ein Polypeptid aus Pilzen mit der biologischen Aktivität einer UMP/CMP-Kinase in *einem in vitro*-System, und

(c) die Gewinnung des Polypeptids aus der Zelle, dem Kulturmedium oder *dem in vitro*-System.

Die so erhaltenen Zellen, enthaltend das erfindungsgemäße Polypeptid oder das so erhaltene gereinigte Polypeptid, sind geeignet, in Verfahren zum Identifizieren von Modulatoren bzw. Inhibitoren der UMP/CMP-Kinase verwendet zu werden.

Gegenstand der vorliegenden Erfindung ist auch die Verwendung von Polypeptiden aus Pilzen, welche zumindest eine biologische Aktivität einer UMP/CMP-Kinase ausüben in Verfahren zum Identifizieren von Inhibitoren eines Polypeptids aus Pilzen mit der Aktivität einer UMP/CMP-Kinase, wobei die Inhibitoren der UMP/CMP-Kinase als Fungizide verwendet werden können. Besonders bevorzugt wird die UMP/CMP-Kinase aus *U. maydis* verwendet.

Fungizide Wirkstoffe, die mit Hilfe einer UMP/CMP-Kinase aus einer bestimmten Pilzspezies gefunden werden, können auch mit UMP/CMP-Kinasen aus anderen Pilzspezies interagieren, wobei die Interaktion mit den unterschiedlichen in diesen Pilzen vorkommenden UMP/CMP-Kinasen nicht immer gleich stark sein muss. Dies erklärt unter anderem die Selektivität wirksamer Substanzen. Die Nutzung der Wirkstoffe, die mit einer spezifischen UMP/CMP-Kinase gefunden wurden als Fungizide auch bei anderen Pilze-Spezies kann darauf zurückgeführt werden, dass sich UMP/CMP-Kinasen aus verschiedenen Pilzspezies sehr nahe stehen und in größeren Bereichen eine ausgeprägte Homologie zeigen. So wird aus Abbildung 1 deutlich, dass eine solche Homologie über beträchtliche Sequenzabschnitte hinweg zwischen *S. cerevisiae, N. crassa, S. pombe* und *U maydis* besteht und damit die Wirkung der mit Hilfe der UMP/CMP-Kinase aus *U. maydis* gefundenen Substanzen nicht auf *U. maydis* beschränkt bleibt.

Gegenstand der vorliegenden Erfindung ist deshalb auch ein Verfahren zum Identifizieren von Fungiziden durch Testen von potentiellen Inhibitoren bzw. Modulatoren der enzymatischen Aktivität der UMP/CMP-Kinase ("Kandidatenverbindungen" oder "Testverbindungen") in einem UMP/CMP-Kinase Hemmtest.

Verfahren, die geeignet sind, Modulatoren, insbesondere Inhibitoren bzw. Antagonisten der erfindungsgemäßen Polypeptide zu identifizieren, beruhen in aller Regel auf der Bestimmung der Aktivität bzw. der biologischen Funktionalität des Polypeptids. Dazu kommen prinzipiell sowohl auf ganzen Zellen beruhende Verfahren (*in vivo* Verfahren) in Frage, wie auch Verfahren, die auf der Verwendung des aus den Zellen isolierten Polypeptids beruhen, das in gereinigter oder teilweise gereinigter Form oder auch als Rohextrakt vorliegen kann. Diese zellfreien *in vitro* Verfahren können ebenso wie *in vivo* Verfahren im Labormaßstab, in bevorzugter Weise aber auch in HTS oder UHTS Verfahren genutzt werden. Im Anschluss an die *in vivo* oder *in vitro* Identifizierung von Modulatoren des Polypeptids können Tests an Pilzkulturen durchgeführt werden, um die fungizide Wirksamkeit der gefundenen Verbindungen zu prüfen.

Viele Testsysteme, die die Prüfung von Verbindungen und natürlichen Extrakten zum Ziel haben, sind bevorzugt auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder teilweise gereinigtes Protein. Sie sind geeignet für eine erste Prüfung, die in erster Linie darauf abzielt, einen möglichen Einfluss einer Substanz auf das Zielprotein zu detektieren. Ist eine solche erste Prüfung erfolgt und eine oder mehrere Verbindungen, Extrakte etc. gefunden, kann die Wirkung solcher Verbindungen im Labor noch gezielter untersucht werden. So kann in einem ersten Schritt die Inhibierung oder Aktivierung des erfindungsgemäßen Polypeptids *in vitro* noch einmal geprüft werden, um im Anschluss daran die Wirksamkeit der Verbindung am Zielorganismus, hier einem oder mehreren pflanzenpathogenen Pilzen, zu testen. Die Verbindung kann dann gegebenenfalls als Ausgangspunkt für die weitere Suche und Entwicklung von fungiziden Verbindungen verwendet werden, die auf der ursprünglichen Struktur basieren, jedoch z.B. hinsichtlich Wirksamkeit, Toxizität oder Selektivität optimiert sind.

Um Modulatoren aufzufinden, kann z.B. ein synthetischer Reaktionsmix (z.B. Produkte der *in vitro* Transkription) oder ein zellulärer Bestandteil, wie eine Membran, ein Kompartiment oder irgendeine andere Präparation, die die erfindungsgemäßen Polypeptide enthält, zusammen mit einem gegebenenfalls markierten Substrat oder Liganden der Polypeptide in Gegenwart und Abwesenheit eines Kandidatenmoleküls inkubiert werden. Die Fähigkeit des Kandidatenmoleküls, die Aktivität der erfindungsgemäßen Polypeptide zu hemmen, wird z.B. erkennbar an einer verringerten Bindung des gegebenenfalls markierten Liganden oder an einer verringerten Umsetzung des gegebenenfalls markierten Substrates. Moleküle, die die biologische Aktivität der erfindungsgemäßen Polypeptide hemmen, sind gute Antagonisten.

Die Detektion der biologischen Aktivität der erfindungsgemäßen Polypeptide kann durch ein so genanntes Reportersystem verbessert werden. Reportersysteme in dieser Hinsicht umfassen, sind aber nicht beschränkt auf colorimetrisch oder fluorimetrisch nachweisbare Substrate, die in ein Produkt umgewandelt werden oder ein Reportergen, das auf Veränderungen der Aktivität oder der Expression der erfindungsgemäßen Polypeptide anspricht oder andere bekannte Bindungstests.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder colorimetrisch, so dass man die Anzahl der Polypeptide, die an einen Liganden gebunden sind oder die eine Umsetzung mitgemacht haben, exakt bestimmen kann. Ebenso kann jedoch die Bindung mittels des gegebenenfalls markierten Substrats, Liganden bzw. Substratanalogen verfolgt werden. Auf diese Weise lässt sich die Effektivität von Antagonisten ermessen.

Eine andere Möglichkeit zur Identifizierung von Substanzen, die die Aktivtät der erfindungsgemäßen Polypeptide modulieren, ist so auch der sogenannten "Scintillation Proximity Assay" (SPA, siehe EP 015473). Dieses Testsystem nutzt die Interaktion eines Polypeptids (z.B. UMP/CMP-Kinase aus *U. maydis*) mit einem radiomarkierten Liganden bzw. Substrat. Das Polypeptid ist dabei an kleine Kügelchen ("Microspheres") oder Perlen ("Beads") gebunden, die mit szintillierenden Molekülen versehen sind. Im Verlauf des Abfalls der Radioaktivität wird die szintillierende Substanz im Kügelchen durch die subatomaren Partikel des radioaktiven Markers angeregt und ein detektierbares Photon emittiert. Die Testbedingungen werden so optimiert, dass nur jene vom Liganden ausgehenden Partikel zu einem Signal führen, die von einem an das erfindungsgemäße Polypeptid gebundenen Liganden ausgehen.

Effekte wie Zelltoxizität werden in diesen *in vitro* Systemen in der Regel ignoriert. Die Testsysteme überprüfen dabei sowohl inhibierende bzw. suppressive Effekte der Substanzen, als auch stimulatorische Effekte. Die Effektivität einer Substanz kann durch konzentrationsabhängige Testreihen überprüft werden. Kontrollansätze ohne Testsubstanzen bzw. ohne Enzym können zur Bewertung der Effekte herangezogen werden.

Durch die anhand der vorliegenden Erfindung verfügbaren, für eine erfindungsgemäße UMP/CMP-Kinase kodierende Nukleinsäuren enthaltenden Wirtszellen, wird die Entwicklung von Testsystemen, die auf Zellen basieren, zur Identifizierung von Substanzen ermöglicht, die die Aktivität der erfindungsgemäßen Polypeptide modulieren.

Eine weitere Möglichkeit zur Identifizierung von Inhibitoren des erfindungsgemäßen Polypeptides beruht auf so genannten *in vivo* oder cell-based Verfahren, die im Prinzip auf den folgenden Schritten basieren: (1) Herstellen von transgenen Organismen, die nach Transformation mit einer erfindungsgemäßen Nukleinsäure in der Lage sind, eine UMP/CMP-Kinase zu exprimieren, (2) Aufbringen einer Testverbindung auf den Organismus aus Schritt (1) und zur Kontrolle auf einen analogen, nicht transformierten Organismus, (3) Bestimmen des Wachstums oder der Überlebensfähigkeit des transgenen und eines nicht-transformierten Organismus nach dem Aufbringen der Testverbindung aus Schritt (2), und (4) Selektion von Testverbindungen, die im Vergleich mit dem Wachstum des transgenen Organismus ein vermindertes Wachstum, eine verminderte Überlebensfähigkeit und/oder verminderte Pathogenität des nicht transgenen Organismus bewirken. Unter einem analogen, nicht transformierten Organismus ist ein Organismus zu verstehen, der als Ausgangsorganismus in Schritt (1) verwendet wurde. Die Transformation kann mit einem erfindungsgemäßen Vektor oder der erfindungsgemäßen Nukleinsäure selbst erfolgen. Als Organismen, die mit der erfindungsgemäßen Nukleinsäure bzw. Vektor transformiert werden, eignen sich bevorzugt Pilze, besonders bevorzugt die eingangs erwähnten phytopathogenen Pilze.

Vorzugsweise handelt es sich bei den zu identifizierenden Modulatoren um kleine organisch-chemische Verbindungen.

Ein Verfahren zum Identifizieren einer Verbindung, die die Aktivität einer UMP/CMP-Kinase aus Pilzen moduliert und die als Fungizid im Pflanzenschutz verwendet werden kann, besteht bevorzugt darin, dass man

a) ein erfindungsgemäßes Polypeptid oder eine Wirtszelle enthaltend dieses Polypeptid mit einer chemischen Verbindung oder mit einem Gemisch von chemischen Verbindungen unter Bedingungen in Kontakt bringt, die die Interaktion einer chemischen Verbindung mit dem Polypeptid erlauben,

b) die Aktivität des erfindungsgemäßen Polypeptids bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, und

c) die chemische Verbindung bestimmt, die die Aktivität des erfindungsgemäßen Polypeptids spezifisch moduliert, und gegebenenfalls

d) die fungizide Wirkung der bestimmten Verbindung *in vivo* prüft.

Besonders bevorzugt wird dabei diejenige Verbindung bestimmt, die die Aktivität des erfindungsgemäßen Polypeptids spezifisch inhibiert. Der Begriff "Aktivität", wie er hier verwendet wird, bezieht sich auf die biologische Aktivität des erfindungsgemäßen Polypeptids.

Ein bevorzugtes Verfahren nutzt die Tatsache aus, dass bei der Reaktion der UMP/CMP-Kinase ADP freigesetzt wird. Die Aktivität bzw. die Ab- oder Zunahme der Aktivität des erfindungsgemäßen Polypeptids kann deswegen durch Kopplung dieser Reaktion mit der enzymatischen Umsetzung des ADP durch die Pyruvatkinase und der darauf folgenden enzymatischen Umsetzung des dabei entstehenden Pyruvats durch die Lactatdehydrogenase erfolgen. Dabei wird die Tatsache genutzt, dass das bei der Reaktion der Pyruvatkinase entstehende Pyruvat von der Lactatdehydrogenase (LDH) unter Verbrauch von NADH zu Lactat umgesetzt wird. Die drei miteinander gekoppelten enzymatischen Reaktionen können beispielhaft wie folgt dargestellt werden:

I. ATP + UMP <=> ADP + UDP     **(UMP/CMP-Kinase)**

II. Phosphoenolpyruvat + ADP + $H^+$ <=> Pyruvat + ATP     **(Pyruvatkinase)**

III. Pyruvat + NADH + $H^+$ <=> Lactat + $NAD^+$     **(Laktatdehydrogenase)**

Das bei der Reaktion der LDH verbrauchte Cosubstrat NADH besitzt bei 340 nm ein Extinktionsmaximum. Der Nachweis des umgesetzten NADH durch Messung der Absorptionsabnahme bei 340 nm bzw. der Fluoreszenzabnahme bei 340 nm (Emmision bei 465 nm) gibt daher Aufschluss über die enzymatische Aktivität der UMP/CMP-Kinase.

Eine geringere bzw. inhibierte Aktivität des erfindungsgemäßen Polypeptids, die zu einer verminderten Entstehung von ADP führt, resultiert letzlich in einem geringeren Verbrauch von NADH. Eine geringere Abnahme der NADH-Konzentration bei Anwesenheit einer Testverbindung im Hemmtest im Vergleich zu einem Ansatz ohne Testverbindung weißt damit auf eine Inhibition der enzymatischen Aktivität der UMP/CMP-Kinase hin. Umgekehrt bedeutet eine stärkere Abnahme der NADH-Konzentration bei Anwesenheit einer Testverbindung im Vergleich zur Kontrolle, dass die Testverbindung eine stimulierende Wirkung auf die enzymaische Aktivität der UMP/CMP-Kinase hat.

Die Messung kann auch in für HTS- oder UHTS-Assays gängigeren Formaten erfolgen, z.B. in Mikrotiterplatten, in denen z.B. ein Gesamtvolumen von 5 bis 50 µl pro Ansatz bzw. pro Well vorgelegt wird und die einzelnen Komponenten in der gewünschten Endkonzentrationen vorliegen (vgl. Beispiel 3). Dabei wird die zu testende, potentiell die Aktivität des Enzyms inhibierende oder aktivierende Verbindung (Kandidaten- oder Testverbindung) z.B. in einer geeigneten Konzentration in Testpuffer enthaltend ATP, UMP bzw. CMP, PEP, NADH sowie die Hilfsenzyme Pyruvatkinase und Laktatdehydrogenase vorgelegt. Dann wird das erfindungsgemäße Polypeptid in Testpuffer zugegeben und die Reaktion damit gestartet. Der Ansatz wird dann z.B. bis zu 30 Minuten bei einer geeigneten Temperatur inkubiert und z.B. die Absorptionsabnahme bei 340 nm gemessen.

Eine weitere Messung erfolgt in einem entsprechenden Ansatz, jedoch ohne Zugabe eines Kandidatenmoleküls und ohne Zugabe eines erfindungsgemäßen Polypeptids (Negativkontrolle). Eine weitere Messung erfolgt wiederum bei Abwesenheit eines Kandidatenmoleküls, jedoch bei Anwesenheit des erfindungsgemäßen Polypeptids (Positivkontrolle). Negativ- und Positivkontrolle ergeben damit die Vergleichswerte zu den Ansätzen bei Anwesenheit eines Kandidatenmoleküls.

Um optimale Bedingungen für ein Verfahren zum Identifizieren von Inhibitoren der UMP/CMP-Kinase bzw. zur Bestimmung der Aktivität der erfindungsgemäßen Polypeptide zu ermitteln, kann es vorteilhaft sein, den jeweiligen $K_m$-Wert des verwendeten erfindungsgemäßen Polypeptids zu bestimmen. Dieser gibt Aufschluss über die bevorzugt zu verwendende Konzentration des bzw. der Substrate. Im Fall der UMP/CMP-Kinase aus *U maydis* konnte ein $K_m$ von 0,12 mM für UMP und ein $K_m$ von 0,06 mM für ATP bestimmt werden (siehe Abbildungen 4A und 4B).

Mit Hilfe der vorstehend beispielhaft beschriebenen Verfahren können Verbindungen identifiziert werden, die die pilzliche UMP/CMP-Kinase inhibieren.

Neben den beispielhaft genannten Verfahren zur Bestimmung der enzymatischen Aktivität einer UMP/CMP-Kinase bzw. der Inhibition dieser Aktivität und zum Identifizieren von Fungiziden können auch andere, z.B. bereits bekannte, Verfahren zur Bestimmung der enzymatischen Aktivität von UMP/CMP-Kinasen verwendet werden, solange diese Verfahren es gestatten, eine Änderung dieser Aktivität bei Anwesenheit eines potentiellen Inhibitors oder Aktivators zu erkennen.

Es kann im Rahmen der vorliegenden Erfindung auch geprüft werden, ob die mit Hilfe eines erfindungsgemäßen Verfahrens identifizierten Inhibitoren einer erfindungsgemäßen UMP/CMP-Kinase geeignet sind, alleine bzw. in einer geeigneten Formulierung Pilze zu schädigen oder zu töten. Die Inhibitoren von pilzlichen UMP/CMP-Kinasen können dann als Fungizide verwendet werden.

In die Kavitäten von Mikrotiterplatten wird dazu z. B. eine methanolische Lösung des zu prüfenden Wirkstoffs, versetzt mit dem Emulgator PS16, pipettiert. Nachdem das Lösungsmittel abgedampft ist, werden je Kavität 200μl Potato-Dextrose-Medium hinzugefügt. Das Medium wird vorher mit einer geeigneten Konzentration von Sporen bzw. Mycel des zu prüfenden Pilzes versetzt. Die resultierenden Konzentrationen des Wirkstoffs betragen 0.05, 0,5, 5 und 50ppm. Die resultierende Konzentration des Emulgators beträgt 300ppm.

Die Platten werden anschließend 3-5 Tage auf einem Schüttler bei einer Temperatur von 20°C inkubiert, bis in der unbehandelten Kontrolle ein ausreichendesWachstum feststellbar ist. Die Auswertung erfolgt photometrisch bei einer Wellenlänge von 620nm. Aus den Messdaten der verschiedenen Konzentrationen wird die Wirkstoffdosis, die zu einer 50 %-igen Hemmung des Pilzwachstums gegenüber der unbehandelten Kontrolle führt ($ED_{50}$), berechnet.

Verbindungen, die mit Hilfe eines erfindungsgemäßen Verfahrens identifiziert werden, und die aufgrund der Inhibition von pilzlichen UMP/CMP-Kinasen eine fungizide Wirkung aufweisen, können somit zur Herstellung von fungiziden Mitteln verwendet werden.

Die identifizierten Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Beim Einsatz der erfindungsgemäßen Verbindungen als Fungizide können die Aufwandmengen je nach Applikation innerhalb größerer Bereiche variiert werden.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z. B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die nachfolgenden Beispiele illustrieren verschiedene Aspekte der vorliegenden Erfindung und sind nicht limitierend auszulegen.

## Beispiele

## Beispiel 1

**Herstellung von *uck1* Knock-Out Mutanten in *U. maydis***

Kultivierung von *U. maydis*

Die Stämme wurden bei 28°C auf PD-, YEPS- oder geeigneten Minimalmedien (Holliday, 1974; Tsukada et al., 1988) angezogen. Die Bildung dikaryotischer Filamente wurde nach dem Auftropfen von Stämmen auf PD-Plattenmedien mit 1 % Charcoal beobachtet (Holliday, 1974). Pathogenitätstests wurden wie beschrieben durchgeführt (Gillessen et al., 1992). Übemachtkulturen der Stämme wurden in einer Konzentration von $4 \times 10^7$ Zellen resuspendiert und jungen Maispflanzen (Gaspe Flint) injiziert bzw. auf getropft. Für jeden Stamm wurden mindestens 25 Pflanzen infiziert und entstehende Tumore nach 14-21 Tagen untersucht.

Herstellung der Knock-Out Kassette

Molekularbiologische Standardmethoden wurden nach *Sambrook et al.,* 1989 durchgeführt. Um *uck*-Nullmutanten herzustellen, wurde die 5' und die 3' Flanke des *uck*-Gens mittels PCR amplifiziert. Als Template diente genomische DNA des Stammes UM518. Für die 5' Flanke (1418bp) wurden die Primer LB2 (Sequenz 5'-cacggcctgagtggccccgagatcaaggaggctatggatc-3') und p 15 (5'-ccgagcgtggattcgagttcc-3') eingesetzt. Für die 3'-Flanke (1393bp) wurden die Primer RB1 (5'-gtgggccatctaggccccttctcaacggcaccagcacc-3') und p13 (5'-ccatcgttgccctaggcactcgcc-3') verwendet. Mit den Primem LB2 und RB1 wurden die Restriktionsstellen *Sfi* I (a) und *Sfi* I (b) eingeführt. Die Amplikons wurden mit *Sfi* I geschnitten und mit dem 1884 bp großen *Sfi* I-Fragment aus dem Vektor pBS-isoliert (HygromycinB-Kassette) ligiert. Durch eine PCR mit den Primern LB1 (5'-cctacgcagacgatccaccacc-3') und RB2 (5'-cgtggatcgcctagaaaaagcgcc-3') wurde die 3851 bp große *uck1* Knock-Out Kassette amplifiziert, die in der nachfolgenden Transformation eingesetzt wurde (Kämper und Schreier, 2001).

Herstellung, von Protoplasten von U *maydis*

50 ml einer Kultur in YEPS Medium wurden bei 28°C bis zu einer Zelldichte von ca. $5 \times 10^7$/ml (OD 0.6 bis 1.0) angezogen und dann für 7 min bei 2500 g (Heraeus, 3500 rpm) in 50 ml Falconröhrchen abzentrifugiert. Das Zellpellet wurde in 25 ml SCS-Puffer (20 mM NaCitrat pH 5.8, 1.0 M Sorbit, (20 mM NaCitrat/1.0 M Sorbit und 20 mM Zitronensäure/1.0 M Sorbit mischen und mit pH-Meter auf pH 5.8 einstellen)) resuspendiert, erneut 7 min bei 2500 g (3500 rpm) zentrifugiert und das Pellet in 2 ml SCS-Puffer, pH 5,8, mit 2,5 mg/ml Novozym 234 resuspendiert. Die Protoplastierung erfolgte bei Raumtemperatur und wurde mikroskopisch alle 5 min verfolgt. Die Protoplasten wurden dann mit 10 ml SCS-Puffer gemischt und bei 1100 g (2300 rpm) für 10 min zentrifugiert, der Überstand wurde verworfen. Das Pellet wurde vorsichtig in 10 ml SCS Puffer resuspendiert und erneut zentrifugiert. Der Waschvorgang mit SCS-Puffer wurde zweimal wiederholt und das Pellet in 10 ml STC-Puffer gewaschen. Schließlich wurde das Pellet in 500 µl kaltem STC-Puffer (10 mM Tris/HCL pH 7.5, 1.0 M Sorbit, 100 mM CaCl$_2$) resuspendiert und auf Eis gehalten. Aliquots können mehrere Monate bei -80°C gelagert werden.

Transformation von *U. maydis*

Die Transformation eines diploiden *U. maydis* Stammes erfolgte nach Schulz *et al.,* 1990. Die Isolierung genomischer *U. maydis* DNA erfolgte wie bei Hoffmann und Winston 1987 beschrieben bzw. nach dem Protokoll der Firma Qiagen (DNeasy-Kit).

Für die Transformation wurden maximal 10 µl DNA (optimal 3-5 µg) in ein 2 ml Eppendorfreaktionsgefäß übertragen und 1 µl Heparin (15 µg/ul) (SIGMA H3125) sowie 50 µl Protoplasten zugegeben. Nach einer Inkubationszeit von 10 min auf Eis wurden 500 µl 40 % (w/w) PEG3350 (SIGMA P3640) in STC (sterilfiltriert) zugesetzt, vorsichtig mit der Protoplastensuspension gemischt und 15 min auf Eis inkubiert. Das Ausplattieren erfolgte auf Gradienten-Platten (unterer Agar: 10 ml YEPS-1,5 % Agar-1M Sorbitol mit Antibiotikum; kurz vor dem Ausplattieren wurde die untere Agarschicht mit 10 ml YEPS- 1,5 % Agar-1M Sorbitol überschichtet, die Protoplasten ausgestrichen und die Platten für 3-4 Tage bei 28°C inkubiert.

Der Nachweis der homologen Rekombination in einen genomischen Lokus von *uck* erfolgte über Standardmethoden (PCR oder Southernanalyse) mittels isolierter genomischer DNA. Hierbei wurde gezeigt, daß die Integration der *uck* Knock-Out Kassette in einen genomischen *uck*-Lokus stattgefunden hat und somit eine Wildtypkopie des *uck*-Gens ersetzt wurde, während die zweite Kopie in diesem diploiden Stamm erhalten blieb. Die auf diese Weise entstandenen heterozygoten uck-Mutanten wurden nachfolgend in den Pathogenitätstest eingesetzt (Gillessen et al., 1992).

Sporenanalyse von *U. maydis*

Aus den im Pathogenitätstest erzeugten Tumoren wurden Sporen isoliert. Im Anschluss wurden die entstehenden Sporidien vereinzelt und phäno- bzw. genotypisch untersucht. Die phänotypische Analyse erfolgte mittels Wachstumsversuchen auf geeigneten Voll- bzw. Minimalmedien (Holliday, 1974; Tsukada et al., 1988). Dabei zeigte sich, das keine von 48 analysierten Sporidien unter selektiven Bedingungen wuchs. Dies war ein erster Hinweis darauf, dass der Phänotyp der *uck*-Nullmutante lethal war. Die genotypischen Untersuchungen erfolgten durch Southern-, bzw.

PCR basierter Analyse der Sporidien. Hierbei wurde gefunden, das unter 96 analysierten Sporidien kein lebensfähiger, haploider Stamm identifiziert werden konnte, in dem nur das *uck-Gen* durch die *uck* Knock-Out Kassette ersetzt war. In den Fällen, in denen die *uck* Knock-Out Kassette homolog in den genomischen Lokus von *uck* integriert war, wurde zusätzlich eine ektopische Kopie des Gens gefunden. Aus diesen Ergebnissen wurde geschlossen, dass der Knock-Out des *uck*-Gens in *Ustilago maydis* zu einem lethalen Phänotyp führt.

**Beispiel 2**

**Klonierung, Expression und Reinigung von *uck* bzw. UCK aus *Ustilago maydis***

Für die Klonierung von *uck* wurde der ORF mittels RT-PCR aus Gesamt-mRNA von *Ustilago maydis* über genspezifische Primer amplifiziert. Die entsprechende DNA, ein Amplikon von 798 bp Länge, wurde in den Vektor pDEST17 (Vektor des "Gateway"-Systems der Firma Invitrogen) kloniert. Das resultierende Plasmid pDEST17-UCK enthält die vollständige kodierende Sequenz von *uck* in einer N-terminalen Fusion mit dem His-Tag, der Bestandteil des Vektors pDEST17 ist. Das UCK Fusionsprotein besitzt eine berechnete Masse von 33 kDa.

Für die heterologe Expression des UCK-Proteins wurde das Plasmid pDEST17-UCK in den *E. coli* Stamm BL21(DE3)pLysS transformiert. 50 ml Selektionsmedium (LB-Medium mit 50 µg/ml Ampicillin und 34 µg/ml Chloramphenicol) wurden mit den *E. coli* Transformanten angeimpft und über Nacht bei 30°C unter Schütteln angezogen. Aus dieser Vorkultur wurde 500 ml Selektionsmedium (LB-Medium mit 50 µg/ml Ampicillin und 34 µg/ml Chloramphenicol) mit einer $OD_{600}$ von 0,1 angeimpft und bei 30°C unter Schütteln inkubiert. Die Induktion der Genexpression erfolgte bei einer $OD_{600}$ von 0,8 bis 1,0 durch Zugabe von IPTG bis zu einer Endkonzentration von 1 mM. Nach einer Induktionszeit von 3,5 h bei 30°C wurden die Zellen durch Zentrifugation geerntet und das Zellpellet bei -80°C gelagert.

Für den Zellaufschluss wurde das Pellet auf Eis aufgetaut, in 4 ml Binding Buffer resuspendiert (0,05 M $NaPO_4$, 0,5 M NaCl, 0,02 M Imidazol, pH 7,4) und anschliessend durch Sonifizierung für 3 min auf Eis aufgeschlossen. Die lösliche Cytoplasmafraktion wurde durch Zentrifugation für 15 min bei 4°C mit 10 000 g erhalten und nach Sterilfiltration durch einen 0,45 µM Filter für die Aufreinigung des exprimierten Proteins verwendet. Die Reinigung erfolgte über Affinitätschromatographie mittels des ÄKTAexplorer FPLC Systems der Firma Amersham unter Verwendung von Hi-Trap Chelating HP 5 ml Säulen nach Angaben des Herstellers. Die Fraktionen des von der Säule eluierten Proteins (Elution Buffer: 0,05 M $NaPO_4$, 0,5 M NaCl, 0,M M Imidazol, 20 % Glycerin, pH 7,4) wurden vereinigt und bei -80°C gelagert.

Beispiel 3

Identifizierung von Modulatoren der UMP/CMP-Kinase in 384-well-MTP in einem gekoppelten Assay

Zur Identifizierung von Modulatoren der UMP/CMP-Kinase wurden 384-well-Mikrotiterplatten von Greiner verwendet.

In die Spalten eins und zwei wurde die Negativ-Kontrolle pipettiert. Diese setzte sich zusammen aus 5 μl 5 % DMSO, 20 μl Substratlösung (50 mM $KPO_4$, pH 7,6; 0,75 mM NADH; 0,375 mM ATP; 0,75 mM PEP; 0,75 mM UMP; 25 mM DTT; 12,5 mM $MgCl_2$; 0,01 % Tween20) und 25 μl Enzymlösung (50 mM $KPO_4$, pH 7,6; 0,1 % BSA; 8 U/ml Pyruvat Kinase; 16 U/ml Lactat Dehydrogenase).

In die Spalten drei und vier wurde die Positiv-Kontrolle pipettiert. Diese setzte sich zusammen aus 5 μl 5 % DMSO, 20 μl Substratlösung (50 mM $KPO_4$, pH 7,6; 0,75 mM NADH; 0,375 mM ATP; 0,75 mM PEP; 0,75 mM UMP; 25 mM DTT; 12,5 mM $MCl_2$; 0,01 % Tween20) und 25 μl Enzymlösung (50 mM $KPO_4$, pH 7,6; 0,1 % BSA; 8 U/ml Pyruvat Kinase; 16 U/ml Laktat Dehydrogenase; 5,1; 0,04 μg/ml UMP/CMP-Kinase).

In die verbleibenden Spalten wurde eine Prüfsubstanz in einer Konzentration von 10 μM in DMSO vorgelegt, wobei zum Verdünnen der Substanz ein Volumen von 5 μl des Testpuffers verwendet wurde. Nach Zugabe von 20 μl Substratlösung (50 mM $KPO_4$, pH 7,6; 0,75 mM NADH; 0,375 mM ATP; 0,75 mM PEP; 0,75 mM UMP; 25 mM DTT; 12,5 mM $MgCl_2$; 0,01 % Tween20) wurden zum Start der Reaktion 25 μl Enzymlösung (50 mM $KPO_4$, pH 7,6; 0,1 % BSA; 8 U/ml Pyruvat Kinase; 16 U/ml Laktat Dehydrogenase; 5,1; 0,04 μg/ml UMP/CMP-Kinase) zugegeben.

Es folgte eine Inkubation bei Raumtemperatur für 30 Minuten. Anschließend wurde das während der Reaktion umgesetzte NADH durch Bestimmung der Absorptionsabnahme bei 340 nm in einem für MTP geeigneten SPECTRAFluor Plus von Tecan gemessen.

## Beispiel 4

### Nachweis der fungiziden Wirkung der identifizierten Inhibitoren der UMP/CMP-Kinase

In die Kavitäten von Mikrotiterplatten wird eine methanolische Lösung des zu prüfenden Wirkstoffs, versetzt mit dem Emulgator PS16, pipettiert. Nachdem das Lösungsmittel abgedampft ist, werden je Kavität 200μl Potato-Dextrose-Medium hinzugefügt. Das Medium wird vorher mit einer geeigneten Konzentration von Sporen bzw. Mycel des zu prüfenden Pilzes versetzt. Die resultierenden Konzentrationen des Wirkstoffs betragen 0.05, 0,5, 5 und 50 ppm. Die resultierende Konzentration des Emulgators beträgt 300 ppm.

Die Platten werden anschließend 3-5 Tage auf einem Schüttler bei einer Temperatur von 20°C inkubiert, bis in der unbehandelten Kontrolle ein ausreichendes Wachstum feststellbar ist.

Die Auswertung erfolgt photometrisch bei einer Wellenlänge von 620nm. Aus den Messdaten der verschiedenen Konzentrationen wird die Wirkstoffdosis, die zu einer 50 %-igen Hemmung des Pilzwachstums gegenüber der unbehandelten Kontrolle führt ($ED_{50}$), berechnet.

### Literatur

1. Amann et al., (1988), Tightly regulated tac promoter vectors useful for the expression of unfused and fused proteins in *Escherichia coli.* Gene 69:301-315.

2. Baldari et al. (1987), A novel leader peptide which allows efficient secretion of a fragment of human interleukin 1 beta in Saccharomyces cerevisiae. Embo J. 6:229-234.

3. Gillissen, B., Bergemann, J., Sandmann, C. Schroeer, M., Bölker, M., und Kahmann, R. (1992), A two-component regulatory system for self/nonself recognition in *Ustilago maydis.* Cell, 68: 647-657.

4. Hoffman, Charles S.; Winston, Fred. A ten-minute DNA preparation from yeast efficiently releases autonomous plasmids for transformation of Escherichia coli. Gene (1987), 57(2-3), 267-72.

5. Hofmann K., Bucher P., Falquet L., Bairoch A. (1999) "The PROSITE database, its status in 1999". *Nucleic Acids Res.* 27, 215.

6. Holliday, R. (1974), *Ustilago maydis.* In King, R. C. (ed), Handbook of Genetics. Plenum, New York, pp. 575-595.

7. Jong, A. et al. (1993), Characteristics, substrate analysis, and intracellular location of *Saccharomyces cerevisiae* UMP kinase. Arch. Biochem. Biophys. 304 (1): 197-204.

8. Kämper und Schreier (2001), Verfahren zur Herstellung von Deletionsmutanten; Deutsche Patentanmeldung Nr. 10 133 926.3.

9. Kaneko, S. et al. (1998), Cloning, sequence analysis and expression of the basidiomycete *Lentinus edodes* gene uck1, encoding UMP-CMP kinase, the homologue of *Saccharomyces cerevisae URA6* gene. Gene 211: 259-266.

10. Kurjan and Herskowitz (1982), Structure of a yeast pheromone gene (MF alpha): a putative alpha-factor precursor contains four tandem copies of mature alpha-factor. Cell 30:933-943.

11. Lottspeich, F., Zorbas H. (Hrsg.). (1998). Bioanalytik. Spektrum Akademischer Verlag, Heidelberg, Berlin.

12. Luckow, V. A. und Summers, M. D. (1988), Trends in the development of baculovirus expression vectors. Bio/ Technology 6, 47-55.

13. Ma, JJ. et al. (1990), Purification and characterization of *Saccharomyces cerevisiae* uridine monophosphate kinase. Journal of Biol. Chem. 256 (31): 19122-19127.

14. Sambrook, J., Fritsch, E., F. und Maniatis, T. (1989), Molecular cloning: A laboratory manual. Cold spring harbor laboratory press, Cold spring harbor, New York.

15. Schulz, B., Banuett, F., Dahl, M., Schlesinger, R., Schäfer, W., Martin, T., Herskowitz, I. und Kahmann, R. (1990), The *b* alleles of *Ustilago maydis,* whose combinations program pathogenic development, code for polypeptides containing a homeodomain-related motif. Cell 60, 295-306.

16. Schultz et al. (1987), Gene 54:113-123.

17. Tsukuda, T. et al. (1988), Isolation and characterization of an autonomously replicating sequence from *Ustilago maydis.* Mol. Cell. Biol. 8: 3703-3709.

18. Yan, H. und Tsai, MD. (1999), Nucleoside monophosphate kinases: structure, mechanism, and substrate specificity. Adv. Enzymol. Relat. Areas Mol. Biol. 73:103-34.

19. Zhou, L. et al., (1998), Cloning, expression in *Escherichia coli,* and characterization of *Arabidopsis thaliana* UMP/CMP kinase. Plant Physiol. 117: 245-254.

**Patentansprüche**

1.  Ein Verfahren zum Identifizieren von fungiziden Verbindungen, **dadurch gekennzeichnet, dass** man

    (a) eine UMP/CMP-Kinase oder eine Wirtszelle, die eine UMP/CMP-Kinase in ausreichender Menge exprimiert, mit einer chemischen Verbindung oder einem Gemisch von chemischen Verbindungen unter Bedingungen, die die Interaktion der chemischen Verbindung mit dem Polypeptid erlauben, in Kontakt bringt,

    (b) die Aktivität der UMP/CMP-Kinase bei Abwesenheit einer chemischen Verbindung mit der Aktivität der UMP/CMP-Kinase bei Anwesenheit einer chemischen Verbindung oder eines Gemisches von chemischen Verbindungen vergleicht, und

    (c) die chemische Verbindung, die die UMP/CMP-Kinase spezifisch inhibiert, bestimmt.

2.  Ein Verfahren gemäß Anspruch 1, **dadurch gekennzeichnet, dass** man die Aktivität der UMP/CMP-Kinase bestimmt indem man

    (a) das bei der Reaktion der UMP/CMP-Kinase entstehende ADP mittels UMP/CMP-Kinase unter Verbrauch von Phosphoenolpyruvat zu Pyruvat und ATP umsetzt,

    (b) das entstehende Pyruvat mittels Laktatdehydrogenase unter Verbrauch von NADH zu Lactat und NAD$^+$ umsetzt, und

    (c) die Änderung der NADH-Konzentration bestimmt.

3.  Ein Verfahren gemäß Anspruch 1 oder 2, **dadurch gekennzeichnet, dass** man eine UMP/CMP-Kinase aus einem pflanzenpathogenen Pilz verwendet.

4.  Ein Verfahren gemäß einem der Ansprüche 1 bis 3, **dadurch gekennzeichnet, dass** man in einem weiteren Schritt die fungizide Wirkung der identifizierten Verbindung testet, indem man sie mit einem Pilz in Kontakt bringt.

**5.** Die Verwendung von Polypeptiden mit der Aktivität einer UMP/CMP-Kinase zum Identifizieren von Fungiziden.

**6.** Ein Verfahren zum Bekämpfen von pflanzenpathogenen Pilzen, **dadurch gekennzeichnet, dass** man einen Inhibitor einer pilzlichen UMP/CMP-Kinase auf den Pilz und/oder seine Umgebung einwirken lässt.

**7.** Eine Nukleinsäure kodierend für eine UMP/CMP-Kinase aus einem pflanzenpathogenen Pilz, **dadurch gekennzeichnet, dass** sie eine Sequenz umfasst, die ausgewählt ist aus:

a) einer Sequenz gemäß SEQ ID NO: 1,

b) Sequenzen, die für ein Polypeptid kodieren, welches die Aminosäuresequenz gemäß SEQ ID NO: 2 umfasst,

c) Sequenzen, die für ein Polypeptid kodieren, welche das Motiv [LIVMFYWCA]-[LIVMFYW](2)-D-G-[FYI] -P-R-x(3)-[NQ] umfassen,

d) Sequenzen, welche an die unter a) und b) definierten Sequenzen bei einer Hybridisierungstemperatur von 42-65°C hybridisieren, und

e) Sequenzen, welche eine zumindest 80 %-ige, bevorzugt eine zumindest 85 %-ige und besonders bevorzugt eine zumindest 90 %-ige Identität mit den unter a) und b) definierten Sequenzen aufweisen.

**8.** Ein DNA-Konstrukt umfassend eine Nukleinsäure gemäß Anspruch 7 und einen heterologen Promotor.

**9.** Einen Vektor umfassend eine Nukleinsäure gemäß Anspruch 7 oder ein DNA-Konstrukt gemäß Anspruch 8.

**10.** Einen Vektor gemäß Anspruch 9, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

**11.** Eine Wirtszelle enthaltend eine Nukleinsäure gemäß Anspruch 7, ein DNA-Konstrukt gemäß Anspruch 8 oder einen Vektor gemäß Anspruch 9 oder 10.

**12.** Eine UMP/CMP-Kinase aus pflanzenpathogenen Pilzen, **dadurch gekennzeichnet, dass** sie eine Sequenz umfasst, die ausgewählt ist aus:

(a) der Sequenz gemäß SEQ ID NO:2,

(b) Sequenzen, welche eine zumindest 80 %-ige, bevorzugt eine zumindest 85 %-ige, besonders bevorzugt eine 90 %-ige und insbesondere bevorzugt eine 95 %-ige Identität mit der unter a) definierten Sequenz haben,

(c) den unter b) angegebenen Sequenzen, welche das Motiv [LIVMFYWCA]-[LIVMFYW](2)-D-G-[FYI]-P-R-x (3)-[NQ] umfassen, und

(d) Fragmenten der unter a) bis c) angegebenen Sequenzen, welche die gleiche biologische Aktivität aufweisen wie die unter a) definierte Sequenz.

```
                    10              20              30
 1   M - - Y N - - - - - - - - - - - - - - - - - - - - - - - - -   N_crassa
 1   M - - Y N - - - - - - - - - - - - - - - - - - - - - - - - -   S_pombe
 1   M T - - - - - - - - - - - - - - - - - - - - - - - - - - - -   S_cerevisiae
 1   M G L F D K L K H K K H E P V E A A P A P A A H I A S A P A   U_maydis


                    40              50              60
 4   - - - - - - - - - - - - - - - - - - - - - - - - V I F V L G G   N_crassa
 4   - - - - - - - - - - - - - - - - - - - - - - - - V I F V L G G   S_pombe
 3   - - - - - - - - A A T T S Q P A F S P D Q V S V I F V L G G   S_cerevisiae
31   A D A E T G A G A V E K D T P R F D S S K V T V V F V L G G   U_maydis


                    70              80              90
11   P G A G K G T Q C D R L A E K F D K F V H I S A G D C L R E   N_crassa
11   P G A G K G T Q C D R L A E K F D K F V H I S A G D C L R E   S_pombe
25   P G A G K G T Q C E K L V K D Y S - F V H L S A G D L L R A   S_cerevisiae
61   P G A G K G T Q C A R L V Q D Y G - F V H L S A G D L L R A   U_maydis


                   100             110             120
41   E Q N R P G S K Y G N L I K E Y I K D G K I V P M E I T I S   N_crassa
41   E Q N R P G S K Y G N L I K E Y I K D G K I V P M E I T I S   S_pombe
54   E Q G R A G S Q Y G E L I K N C I K E G Q I V P Q E I T L A   S_cerevisiae
90   E Q Q R P G S Q Y G A M I A D Y I K E G K I V P M E V T V A   U_maydis


                   130             140             150
71   L L E T K M K E C H D K - - - - - - - - - - - - - - - - - -   N_crassa
71   L L E T K M K E C H D K - - - - - - - - - - - - - - - - - -   S_pombe
84   L L R N A I S D N V K A N - - - - - - - - - - - - - - - - -   S_cerevisiae
120  L L S N A I A E A L S K Q A T T E A D H S I P E E H K L K W   U_maydis


                   160             170             180
83   - - G I D K F L I D G F P R E M D Q C E G F E K S V C P A K   N_crassa
83   - - G I D K F L I D G F P R E M D Q C E G F E K S V C P A K   S_pombe
97   - - - K H K F L I D G F P R K M D Q A I S F E R D I V E S K   S_cerevisiae
150  S D G K G R F L V D G F P R K M D Q A I K F D E S V C E S K   U_maydis


                   190             200             210
111  F A L Y F R C G Q E T M L K R L I H R G K T S G R S D D N I   N_crassa
111  F A L Y F R C G Q E T M L K R L I H R G K T S G R S D D N I   S_pombe
124  F I L F F D C P E D I M L E R L L E R G K T S G R S D D N I   S_cerevisiae
180  F V L F L Q C S E E V M L E R L L E R G K T S G R A D D N I   U_maydis


                   220             230             240
141  E S I K K R F V T Y T K A S M P V V E Y L K S Q N R L I T I   N_crassa
141  E S I K K R F V T Y T K A S M P V V E Y L K S Q N R L I T I   S_pombe
154  E S I K K R F N T F K E T S M P V I E Y F E T K S K V V R V   S_cerevisiae
210  E S I K K R F Q T F V E T S M P V V D Y Y R K Q D R V V E V   U_maydis


                   250             260
171  D A E Q D P D A V F E D T V K A L Q P Y L                     N_crassa
171  D A E Q D P D A V F E D T V K A L Q P Y L                     S_pombe
184  R C D R S V E D V Y K D V Q D A I R D S L                     S_cerevisiae
240  D S I K T V D Q V Y A E I K E A M D R T F A K L E Q           U_maydis
```

**Abbildung 1**

**Abbildung 2**

**Abbildung 3**

**Abbildung 4**

**Abbildung 5**

**Europäisches Patentamt**

## EUROPÄISCHER TEILRECHERCHENBERICHT

der nach Regel 45 des Europäischen Patent-übereinkommens für das weitere Verfahren als europäischer Recherchenbericht gilt

Nummer der Anmeldung

EP 04 02 7608

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X | WO 02/06512 A (BASF AKTIENGESELLSCHAFT) 24. Januar 2002 (2002-01-24) * Seite 7, Absatz 3 * * Seite 12, Absatz 3 - Absatz 4 * ----- | 1-5 | C12N15/54 C12N9/12 C12N1/21 G01N33/68 |
| X | WO 02/086090 A (ELITRA PHARMACEUTICALS, INC; JIANG, BO; TISHKOFF, DANIEL; ZAMUDIO, CAR) 31. Oktober 2002 (2002-10-31) | 7-12 | |
| A | see gene #355 (SEQ ID NO: 1355 and 3355) * Seite 4, Absatz 1; Ansprüche 3-8,10,14-16,28,29 * * Seite 36, Absätze 4,5 * * Seite 45, Zeilen 3-20 * * Seite 49, Absatz 4 - Seite 54, Absatz 2 * * Seite 95, Absatz 3 * * Seite 98, Absatz 2 * * Seite 109, Absatz 2 - Seite 110, Absatz 2 * ----- -/-- | 4 | |

| RECHERCHIERTE SACHGEBIETE (Int.Cl.7) |
|---|
| C12N G01N |

### UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| Den Haag | 28. Februar 2005 | Oderwald, H |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

| | Europäisches Patentamt | **EUROPÄISCHER TEILRECHERCHENBERICHT** | **Nummer der Anmeldung**<br><br>EP 04 02 7608 |
|---|---|---|---|

| **EINSCHLÄGIGE DOKUMENTE** | | | **KLASSIFIKATION DER ANMELDUNG** (Int.Cl.7) |
|---|---|---|---|
| Kategorie | Kennzeichnung des Dokuments mit Angabe, soweit erforderlich der maßgeblichen Teile | Betrifft Anspruch | |
| D,X | KANEKO S ET AL: "Cloning, sequence analysis and expression of the basidiomycete Lentinus edodes gene uck1, encoding UMP-CMP kinase, the homologue of Saccharomyces cerevisae URA6 gene" GENE: AN INTERNATIONAL JOURNAL ON GENES AND GENOMES, ELSEVIER SCIENCE PUBLISHERS, BARKING, GB, Bd. 211, Nr. 2, 12. Mai 1998 (1998-05-12), Seiten 259-266, XP004120588 ISSN: 0378-1119 | 7-12 | |
| A | * Seite 260, rechte Spalte - Seite 261, rechte Spalte; Abbildungen 2-4 *<br>----- | 2 | **RECHERCHIERTE SACHGEBIETE** (Int.Cl.7) |
| X | DATABASE EMBL [Online] 8. Oktober 2003 (2003-10-08), "D48_G08 Filamentous Forced Diploid Ustilago maydis cDNA 3', mRNA sequence." XP002319387 gefunden im EBI accession no. EM_EST:CF642196 Database accession no. CF642196 * Zusammenfassung *<br>----- | 7-11 | |

EPO FORM 1503 03.82 (P04C12)

Europäisches
Patentamt

**UNVOLLSTÄNDIGE RECHERCHE
ERGÄNZUNGSBLATT C**

Nummer der Anmeldung

EP 04 02 7608

Nicht recherchierte Ansprüche:
      6

Grund für die Beschränkung der Recherche:

Der geltende Patentanspruch 6 bezieht sich auf ein Produkt/ eine Verbindung, charakterisiert durch eine erstrebenswerte Eigenheit oder Eigenschaft, nämlich einen "Inhibitor einer pilzlichen UMP/CMP-Kinase". Die Patentansprüche umfassen daher alle Produkte/Verbindungen, die diese Eigenheit oder Eigenschaft aufweisen, wohingegen die Patentanmeldung Stütze durch die Beschreibung im Sinne von Art. 83 EPÜ keine solchen Produkte/ Verbindungen liefert. Im vorliegenden Fall fehlen den Patentansprüchen die entsprechende Stütze bzw. der Patentanmeldung die nötige Offenbarung in einem solchen Masse, dass eine sinnvolle Recherche über den gesamten erstrebten Schutzbereich unmöglich erscheint. Desungeachtet fehlt den Patentansprüchen auch die in Art. 84 EPÜ geforderte Klarheit, nachdem in ihnen versucht wird, das Produkt/ die Verbindung über das jeweils erstrebte Ergebnis zu definieren. Auch dieser Mangel an Klarheit ist dergestalt, dass er eine sinnvolle Recherche über den gesamten erstrebten Schutzbereich unmöglich macht. Daher wurde keine Recherche für diesen Anspruch durchgeführt.

**EP 1 538 212 A1**

**ANHANG ZUM EUROPÄISCHEN RECHERCHENBERICHT**
**ÜBER DIE EUROPÄISCHE PATENTANMELDUNG NR.**                    EP 04 02 7608

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-02-2005

| Im Recherchenbericht angeführtes Patentdokument | | Datum der Veröffentlichung | Mitglied(er) der Patentfamilie | | Datum der Veröffentlichung |
|---|---|---|---|---|---|
| WO 0206512 | A | 24-01-2002 | AU | 1041702 A | 30-01-2002 |
| | | | CA | 2416039 A1 | 24-01-2002 |
| | | | WO | 0206512 A2 | 24-01-2002 |
| | | | EP | 1301787 A2 | 16-04-2003 |
| | | | US | 2002058244 A1 | 16-05-2002 |
| WO 02086090 | A | 31-10-2002 | CA | 2445179 A1 | 31-10-2002 |
| | | | EP | 1390468 A2 | 25-02-2004 |
| | | | WO | 02086090 A2 | 31-10-2002 |
| | | | US | 2003119013 A1 | 26-06-2003 |

EPO FORM P0461

Für nähere Einzelheiten zu diesem Anhang : siehe Amtsblatt des Europäischen Patentamts, Nr.12/82

32